# EUROPEAN PATENT SPECIFICATION

(11) **EP 2 323 693 B1**
(45) Date of publication and mention of the grant of the patent: **08.11.2017**
(21) Application number: 08822306.0
(22) Date of filing: 12.09.2008
(51) Int. Cl.: A61K 47/10, A61K 47/12, A61K 47/14, A61K 47/34, A61K 47/36, A61K 9/127, A61K 31/00, A61K 31/7088, A61K 31/337

(54) **DRUG CARRIERS**
ARZNEIMITTELTRÄGER
VECTEURS DE MÉDICAMENT

(30) Priority: 30.07.2008 US 84939 P; 30.07.2008 US 84947 P; 30.07.2008 US 84955 P; 30.07.2008 US 84964 P; 30.07.2008 US 84968 P; 30.07.2008 US 84977 P
(43) Date of publication of application: 25.05.2011
(73) Proprietor: Nitto Denko Corporation, Ibaraki-shi, Osaka 567-8680 (JP)
(72) Inventor: NIITSU, Yoshiro, Hokkaido (JP); YU, Lei, Carlsbad, CA 92008 (US); ZHAO, Gang, Vista CA 92081 (US); VAN, Sang, San Diego CA 92111 (US); CHEN, Fu, Carlsbad CA 92010 (US)
(74) Representative: Potter Clarkson LLP
(86) International application number: PCT/US2008/076295
(87) International publication number: WO 2010/014117

(56) References cited:
- EP-A- 0 932 399
- WO-A-97/33618
- WO-A-02/092600
- US-A1- 2002 041 898
- US-A1- 2003 161 791

## Description

### RELATED APPLICATION INFORMATION

### BACKGROUND

### Field

Disclosed herein are compositions and methods related to the fields of organic chemistry, pharmaceutical chemistry, biochemistry, molecular biology and medicine. In particular, embodiments disclosed herein relate to compositions and compositions for use in methods for delivering an active agent into a cell, and to compositions for use in the treatment and alleviation of diseases and disorders characterized by fibrosis.

### Description of the Related Art

Fibrosis, or the development of excess fibrous connective tissue within the body, has been associated with a number of diseases and disorders such as hepatic fibrosis, pancreatic fibrosis, vocal cord scarring, and numerous forms of cancer.

Various approaches have been taken in an attempt to inhibit fibrosis in an organ or tissue. One approach can be to inhibit the activation of one or more stellate cells, wherein activation of such cells is characterized by an increased production of extracellular matrix (ECM). Other approaches may relate to inhibiting the production of collagen, such as by promoting collagen degradation or controlling collagen metabolism. It may be difficult, however, to target a particular organ or tissue in need thereof.

### SUMMARY

The invention provides a therapeutic composition comprising a carrier selected from the group consisting of a non-cationic polymeric carrier, a liposome carrier, a dendritic carrier, a nanomaterial carrier, a biostructural carrier and a micelle carrier, a targeting agent operatively associated with the carrier, wherein the targeting agent includes a retinoid, and a therapeutic agent operatively associated with the carrier, wherein the therapeutic agent exhibits a therapeutic activity upon delivery to a target organ or tissue, and wherein the therapeutic activity is selected from inhibiting fibrosis within the target organ or tissue and inhibiting the growth of a cancer cell within the target organ or tissue.

Some embodiments relate to a therapeutic composition as described herein, and further including at least one selected from a pharmaceutically acceptable excipient and a diluent.

Some embodiments provide a therapeutic composition for use in a method for treating a condition characterized at least on part by abnormal fibrosis that can include administering a therapeutically effective amount of a therapeutic composition described herein to a subject in need thereof.

Other embodiments provide a therapeutic composition described herein for use in treating a condition characterized at least on part by abnormal fibrosis.

Still other embodiments provide a therapeutic composition described herein for treating a condition characterized at least on part by abnormal fibrosis.

These and other embodiments are described in greater detail below.

### BRIEF DESCRIPTION OF THE DRAWINGS

Figure 1 illustrates a reaction scheme for the preparation of a non-cationic polymeric carrier that includes poly-L-glutamic acid and a retinol.
Figure 2 illustrates a reaction scheme for the preparation of a non-cationic polymeric carrier that includes poly-(γ-L-glutamylglutamine) and a retinol.
Figure 3 illustrates a reaction scheme for the preparation of a therapeutic composition that includes poly-L-glutamic acid, a retinol, and paclitaxel.
Figure 4 illustrates a reaction scheme for the preparation of a therapeutic composition that includes poly-(γ-L-glutamylglutamine), a retinol, and paclitaxel.
Figure 5 illustrates an exemplary liposome carrier.
Figure 6 illustrates two exemplary dendritic carriers, respectively, a dendrimer and a dendron.
Figure 7 illustrates an exemplary micelle carrier.
Figure 8 is a graph illustrating the cell uptake of a Texas Red-non-cationic polymeric carrier-retinoid complex as compared to a Texas Red-non-cationic polymeric carrier-cholesterol complex.
Figure 9 is a graph illustrating the cell uptake of a Texas Red-Dextran-retinoid complex as compared to a Texas Red-Dextran-oleic acid complex.

### DETAILED DESCRIPTION

Unless defined otherwise, all technical and scientific terms used herein have the same meaning as is commonly understood by one of ordinary skill in the art.

As used herein, the term "carrier" may be used to refer to various types of substances, including a non-cationic polymeric carrier, a liposome carrier, a dendritic carrier, a nanomaterial carrier, a biostructural carrier, or a micelle carrier. A carrier can be operatively associated with one or more agents, e.g., a therapeutic agent and/or a targeting agent. In this context, "operatively associated" refers to an electronic interaction between the carrier and the agent(s). Such interaction may take the form of a chemical bond, including, but not limited to, a covalent bond, a polar covalent bond, an ionic bond, an electrostatic association, a coordinate covalent bond, an aromatic bond, a hydrogen bond, a dipole, or a van der Waals interaction. Those of ordinary skill in the art understand that the relative strengths of such interactions may vary widely.

A carrier is a substance that facilitates the transport of the one or more agents with which it is operatively associated from one part of the body to a target cell or tissue and/or into a target cell or tissue. The carrier can be electronically charged (e.g., negatively-charged or positively-charged) or electronically neutral. Those skilled in the art will appreciate that, in determining whether the carrier is cationic, anionic, or electronically neutral, any targeting agent and/or therapeutic agent that is operatively associated with the polymeric carrier is not considered to be part of the carrier. In other words, any charge carried by any targeting agent and/or therapeutic agent operatively associated with the carrier is ignored when determining whether the carrier is cationic, anionic, or electronically neutral.

The term "non-cationic polymeric carrier" as used herein refers to an anionic (i.e., negatively-charged) or electronically neutral polymer that may be operatively associated with one or more agents.

The term "lipid" as used herein refers to any fat-soluble (i.e., lipophilic) molecule. A lipid may include, but is not limited to, an oil, wax, sterol, monoglyceride, diglyceride, triglyceride, and phospholipid.

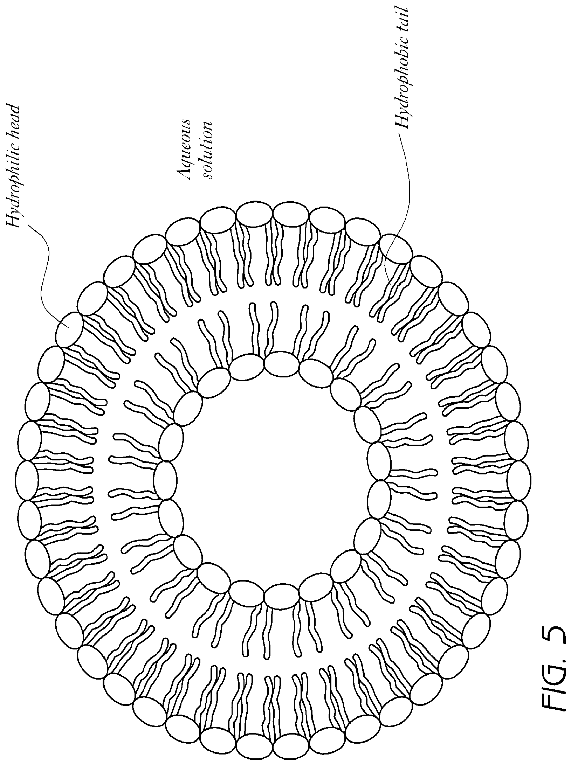

A "liposome carrier" refers to a lipid bilayer structure that contains lipids attached to polar, hydrophilic groups, forming a substantially closed structure in aqueous media that can be operatively associated with one or more agents. An example of a liposome carrier is shown in Figure 5. A liposome carrier may be comprised of a single lipid bilayer (i.e., unilamellar) or it may comprised of two or more concentric lipid bilayers (i.e., multilamellar). A liposome carrier can be approximately spherical or ellipsoidal in shape.

The term "dendritic carrier" refers to a dendrimer, dendron or derivatives thereof that can be operatively associated with one or more agents.

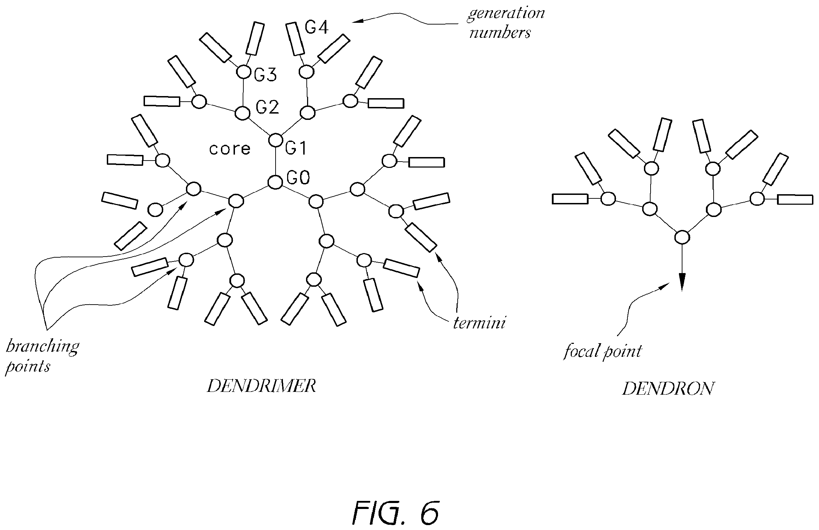

The term "dendrimer" refers to a macromolecule having a core and having multiple shells of branching structures emanating from the core. The term "dendron" is a type of dendrimer having branches that emanate from a focal point. Pictorial examples of a dendrimer and a dendron are shown in Figure 6. For both a dendrimer and a dendron, the branches can be connected to the core directly or through a linking group. A dendrimer typically includes multiple shells or "generations", e.g., G1, G2, G3, etc. as illustrated in Figure 6. A repeated reaction sequence is often used to add each generation to the dendrimer.

The shape and size of a dendritic carrier can vary. In some instances, the dendritic carrier can be approximately spherical or globular in shape. Furthermore, the dendritic carrier can have a diameter in the range of about 15 angstroms (Å) to about 250 Å, with a corresponding range of molecular weights, e.g., from about 500 Daltons to about 2 million Daltons. Dendrimers can be obtained commercially from various sources (e.g., Dendritech, Midland, Michigan) or synthesized by methods known to those skilled in the art.

The term "nanomaterial carrier" as used herein refers to a material that has a longest dimension that is in the range of approximately 100 nm to approximately 1 nm and that can be operatively associated with one or more agents. Exemplary nanomaterial carriers include nanoparticles, nanopolymers, and nanospheres.

"Nanoparticle" refers to a particle that is approximately 100 nm to approximately 1 nm in all dimensions. A nanoparticle can have any shape and any morphology. Examples of nanoparticles include nanopowders, nanoclusters, nanocrystals, nanospheres, nanofibers, and nanotubes.

"Nanopolymer" refers to a polymer that upon polymerization assembles to form a nanoparticle, such as, e.g., a nanorod, nanofiber, or nanosphere.

"Nanosphere" refers to a type of nanoparticle that is approximately spherical in shape. In some instances, a nanosphere may have a hollow core within which one or more agents can be operatively associated.

"Fullerene" refers to an allotrope of carbon molecules that includes, but is not limited to, a spherical fullerene (e.g., C₆₀), a carbon nanotube, fullerene derivatives, and nanotube derivatives. Depending on the size of the fullerene, it may be a nanoparticle.

"Microparticle" refers to a particle that has a size that in the range of approximately 100 nm to approximately 1000 nm in all dimensions. A microparticle can have any shape and any morphology.

The term "biostructural carrier" as used herein refers to a polymer or compound in which the majority of units of the biostructural carrier are amino acids and/or saccharides, and that is operatively associated with one or more agents. Examples of biostructural carriers include sugars, proteins and peptides, as well as semisynthetic derivatives thereof.

The term "sugar" as used herein refers to monosaccharides, oligosaccharides and polysaccharides. A "polysaccharide" is a polymer comprised of recurring monosaccharide units joined by glycosidic bonds. An "oligosaccharide" is a polysaccharide comprised of 2-10 monosaccharide units joined by glycosidic bonds. A sugar can be naturally occurring or synthetic. Examples of sugars include, but are not limited to, glucose (dextrose), fructose, galactose, xylose, ribose, sucrose, cellulose, cyclodextrin and starch.

The term "cyclodextrin" refers to a cyclic polysaccharide that is composed of recurring glucopyranose units attached by α-(1,4) glycosidic bonds. A cyclodextrin may be an oligosaccharide. Exemplary cyclodextrins include α-cyclodextrin, β-cyclodextrin, and γ-cyclodextrin. A cyclodextrin may be unsubstituted or substituted.

The term "protein" as used herein refers to a natural or synthetic compound comprised of 50 or more amino acid units joined by peptide bonds. A protein can be an amino acid chain that is folded into a three-dimensional structure, e.g., tertiary structure. The term "peptide" as used herein refers to a natural or synthetic compound comprised of 2-49 amino acid units joined by peptide bonds. A peptide can have a linear or folded conformation. The amino acids present in a protein and a peptide can be naturally occurring and/or non-naturally occurring.

"Albumin" refers to a class of water-soluble proteins that coagulates upon heating. Albumin can be commonly found in, e.g., blood serum, milk, and/or egg.

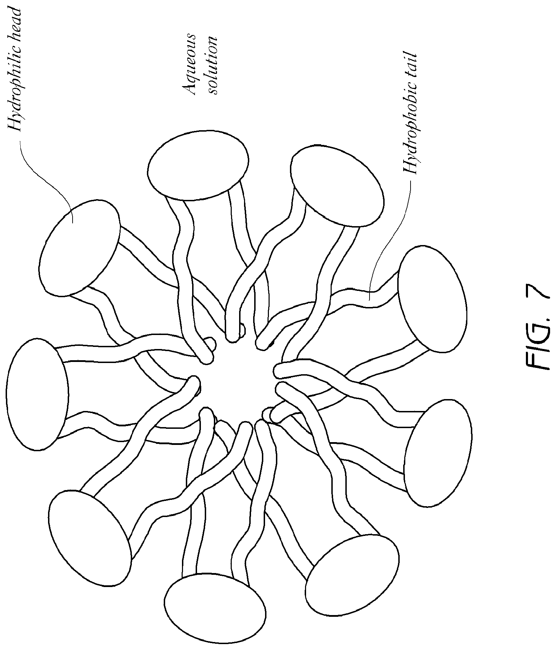

The term "micelle carrier" refers to an aggregate of amphiphilic molecules that may be operatively associated with one or more agents. A micelle carrier can have either a hydrophobic core or a hydrophilic core. An example of a micelle carrier is shown in Figure 7. The micelle carrier may have various shapes including approximately spherical.

The term "targeting agent" refers to a compound that exhibits selectivity for a particular target organ or tissue. A targeting agent is capable of directing a composition, with which it is operatively associated, to a particular target organ or tissue. A targeting agent can be operatively associated with a carrier and/or at least one other compound.

A "retinoid" is a member of the class of compounds consisting of four isoprenoid units joined in a head-to-tail manner, see G. P. Moss, "Biochemical Nomenclature and Related Documents," 2nd Ed. Portland Press, pp. 247-251 (1992). "Vitamin A" is the generic descriptor for retinoids exhibiting qualitatively the biological activity of retinol. As used herein, retinoid refers to natural and synthetic retinoids including first generation, second generation, and third generation retinoids. Examples of naturally occurring retinoids include, but are not limited to, (1) 11-*cis*-retinal, (2) all-*trans* retinol, (3) retinyl palmitate, (4) all-*trans* retinoic acid, and (5) 13-*cis*-retinoic acids. Furthermore, the term "retinoid" encompasses retinols, retinals, and retinoic acids.

The term "therapeutic" refers to the alleviation, prevention, or inhibition of any undesired signs or symptoms of a disease or condition, to any extent. Such undesired signs may include those that worsen the subject's overall feeling of well-being or appearance. This term does not necessarily indicate total cure or abolition of the disease or condition. A "therapeutic agent" is a compound that, upon administration to a mammal in a therapeutically effective amount, provides a therapeutic benefit to the mammal. A therapeutic agent may be referred to herein as a drug. Those skilled in the art will appreciate that the term "therapeutic agent" is not limited to drugs that have received regulatory approval. A "therapeutic agent" can be operatively associated with at least one carrier and/or other agent.

"Fibrosis" is used herein in its ordinary sense and refers to the development of fibrous scar-like connective tissue in an organ or tissue as part of a reparative or reactive process. "Abnormal fibrosis" refers to the development of fibrous scar-like connective tissue in an organ or tissue to an extent that it impairs the function of the organ or tissue.

As used herein, "linker" and "linking group" refer to one or more atoms that connect one chemical moiety to another chemical moiety. Examples of linking groups include relatively low molecular weight groups such as amide, ester, carbonate and ether, as well as higher molecular weight linking groups such as polyethylene glycol (PEG).

As used herein, "Cₘ to Cₙ" in which "m" and "n" are integers refers to the number of carbon atoms in an alkyl, alkenyl or alkynyl group or the number of carbon atoms in the ring of a cycloalkyl, cycloalkenyl, cycloalkynyl, aryl, heteroaryl or heteroalicyclyl group. That is, the alkyl, alkenyl, alkynyl, ring of the cycloalkyl, ring of the cycloalkenyl, ring of the cycloalkynyl, ring of the aryl, ring of the heteroaryl or ring of the heteroalicyclyl can contain from "m" to "n", inclusive, carbon atoms. Thus, for example, a "C₁ to C₄ alkyl" group refers to all alkyl groups having from 1 to 4 carbons, that is, CH₃-, CH₃CH₂-, CH₃CH₂CH₂-, (CH₃)₂CH-, CH₃CH₂CH₂CH₂-, CH₃CH₂CH(CH₃)- and (CH₃)₃C-. If no "m" and "n" are designated with regard to an alkyl, alkenyl, alkynyl, cycloalkyl, cycloalkenyl, cycloalkynyl, aryl, heteroaryl or heteroalicyclyl group, the broadest range described in these definitions is to be assumed.

As used herein, "alkyl" refers to a straight or branched hydrocarbon chain fully saturated (no double or triple bonds) hydrocarbon group. The alkyl group may have 1 to 50 carbon atoms (whenever it appears herein, a numerical range such as "1 to 50" refers to each integer in the given range; *e.g*., "1 to 50 carbon atoms" means that the alkyl group may consist of 1 carbon atom, 2 carbon atoms, 3 carbon atoms, *etc*., up to and including 50 carbon atoms, although the present definition also covers the occurrence of the term "alkyl" where no numerical range is designated). The alkyl group may also be a medium size alkyl having 1 to 30 carbon atoms. The alkyl group could also be a lower alkyl having 1 to 5 carbon atoms. The alkyl group of the compounds may be designated as "C₁-C₄ alkyl" or similar designations. By way of example only, "C₁-C₄ alkyl" indicates that there are one to four carbon atoms in the alkyl chain, i.e., the alkyl chain is selected from the group consisting of methyl, ethyl, propyl, iso-propyl, n-butyl, iso-butyl, sec-butyl, and t-butyl. Typical alkyl groups include, but are in no way limited to, methyl, ethyl, propyl, isopropyl, butyl, isobutyl, tertiary butyl, pentyl, hexyl and the like.

The alkyl group may be substituted or unsubstituted. When substituted, the substituent group(s) is(are) one or more group(s) individually and independently selected from alkenyl, alkynyl, cycloalkyl, cycloalkenyl, cycloalkynyl, aryl, heteroaryl, heteroalicyclyl, aralkyl, heteroaralkyl, (heteroalicyclyl)alkyl, hydroxy, protected hydroxyl, alkoxy, aryloxy, acyl, ester, mercapto, cyano, halogen, carbonyl, thiocarbonyl, O-carbamyl, N-carbamyl, O-thiocarbamyl, N-thiocarbamyl, C-amido, N-amido, S-sulfonamido, N-sulfonamido, C-carboxy, protected C-carboxy, O-carboxy, isocyanato, thiocyanato, isothiocyanato, nitro, silyl, sulfenyl, sulfinyl, sulfonyl, haloalkyl (mono-, di- and tri-substituted haloalkyl), haloalkoxy (mono-, di- and tri-substituted haloalkoxy), trihalomethanesulfonyl, trihalomethanesulfonamido, and amino, including mono- and di-substituted amino groups, and the protected derivatives thereof.

As used herein, "alkenyl" refers to an alkyl group that contains in the straight or branched hydrocarbon chain one or more double bonds. An alkenyl group may be unsubstituted or substituted. When substituted, the substituent(s) may be selected from the same groups disclosed above with regard to alkyl group substitution unless otherwise indicated.

As used herein, "alkynyl" refers to an alkyl group that contains in the straight or branched hydrocarbon chain one or more triple bonds. An alkynyl group may be unsubstituted or substituted. When substituted, the substituent(s) may be selected from the same groups disclosed above with regard to alkyl group substitution unless otherwise indicated.

As used herein, "aryl" refers to a carbocyclic (all carbon) monocyclic or multicyclic aromatic ring system that has a fully delocalized pi-electron system. Examples of aryl groups include, but are not limited to, benzene, naphthalene and azulene. The ring of the aryl group may have 5 to 50 carbon atoms. The aryl group may be substituted or unsubstituted. When substituted, hydrogen atoms are replaced by substituent group(s) that is(are) one or more group(s) independently selected from alkyl, alkenyl, alkynyl, cycloalkyl, cycloalkenyl, cycloalkynyl, aryl, heteroaryl, heteroalicyclyl, aralkyl, heteroaralkyl, (heteroalicyclyl)alkyl, hydroxy, protected hydroxy, alkoxy, aryloxy, acyl, ester, mercapto, cyano, halogen, carbonyl, thiocarbonyl, O-carbamyl, N-carbamyl, O-thiocarbamyl, N-thiocarbamyl, C-amido, N-amido, S-sulfonamido, N-sulfonamido, C-carboxy, protected C-carboxy, O-carboxy, isocyanato, thiocyanato, isothiocyanato, nitro, silyl, sulfenyl, sulfinyl, sulfonyl, haloalkyl (mono-, di- and tri-substituted haloalkyl), haloalkoxy (mono-, di- and tri-substituted haloalkoxy), trihalomethanesulfonyl, trihalomethanesulfonamido, and amino, including mono- and di-substituted amino groups, and the protected derivatives thereof, unless the substituent groups are otherwise indicated.

As used herein, "heteroaryl" refers to a monocyclic or multicyclic aromatic ring system (a ring system with fully delocalized pi-electron system) that contain(s) one or more heteroatoms, that is, an element other than carbon, including but not limited to, nitrogen, oxygen and sulfur. The ring of the heteroaryl group may have 5 to 50 atoms. The heteroaryl group may be substituted or unsubstituted. Examples of heteroaryl rings include, but are not limited to, furan, furazan, thiophene, benzothiophene, phthalazine, pyrrole, oxazole, benzoxazole, 1,2,3-oxadiazole, 1,2,4-oxadiazole, thiazole, 1,2,3-thiadiazole, 1,2,4-thiadiazole, benzothiazole, imidazole, benzimidazole, indole, indazole, pyrazole, benzopyrazole, isoxazole, benzoisoxazole, isothiazole, triazole, benzotriazole, thiadiazole, tetrazole, pyridine, pyridazine, pyrimidine, pyrazine, purine, pteridine, quinoline, isoquinoline, quinazoline, quinoxaline, cinnoline, and triazine. A heteroaryl group may be substituted or unsubstituted. When substituted, hydrogen atoms are replaced by substituent group(s) that is(are) one or more group(s) independently selected from alkyl, alkenyl, alkynyl, cycloalkyl, cycloalkenyl, cycloalkynyl, aryl, heteroaryl, heteroalicyclyl, aralkyl, heteroaralkyl, (heteroalicyclyl)alkyl, hydroxy, protected hydroxy, alkoxy, aryloxy, acyl, ester, mercapto, cyano, halogen, carbonyl, thiocarbonyl, O-carbamyl, N-carbamyl, O-thiocarbamyl, N-thiocarbamyl, C-amido, N-amido, S-sulfonamido, N-sulfonamido, C-carboxy, protected C-carboxy, O-carboxy, isocyanato, thiocyanato, isothiocyanato, nitro, silyl, sulfenyl, sulfinyl, sulfonyl, haloalkyl (mono-, di- and tri-substituted haloalkyl), haloalkoxy (mono-, di- and tri-substituted haloalkoxy), trihalomethanesulfonyl, trihalomethanesulfonamido, and amino, including mono- and di-substituted amino groups, and the protected derivatives thereof.

As used herein, "cycloalkyl" refers to a completely saturated (no double bonds) mono- or multi- cyclic hydrocarbon ring system. When composed of two or more rings, the rings may be joined together in a fused, bridged or spiro-connected fashion. Cycloalkyl groups may range from C₃ to C₁₀, in other embodiments it may range from C₃ to C₈. A cycloalkyl group may be unsubstituted or substituted. Typical cycloalkyl groups include, but are in no way limited to, cyclopropyl, cyclobutyl, cyclopentyl, cyclohexyl, and the like. If substituted, the substituent(s) may be an alkyl or selected from those substituents indicated above with respect to substitution of an alkyl group unless otherwise indicated.

As used herein, "cycloalkenyl" refers to a cycloalkyl group that contains one or more double bonds in the ring although, if there is more than one, the double bonds cannot form a fully delocalized pi-electron system in the ring (otherwise the group would be "aryl," as defined herein). When composed of two or more rings, the rings may be connected together in a fused, bridged or spiro-connected fashion. A cycloalkenyl group may be unsubstituted or substituted. When substituted, the substituent(s) may be an alkyl or selected from the substituents disclosed above with respect to alkyl group substitution unless otherwise indicated.

As used herein, "cycloalkynyl" refers to a cycloalkyl group that contains one or more triple bonds in the ring. When composed of two or more rings, the rings may be joined together in a fused, bridged or spiro-connected fashion. A cycloalkynyl group may be unsubstituted or substituted. When substituted, the substituent(s) may be an alkyl or selected from the substituents disclosed above with respect to alkyl group substitution unless otherwise indicated.

As used herein, "heterocyclyl" and "heteroalicyclyl" refer to a stable 3- to 18 membered ring which consists of carbon atoms and from one to five heteroatoms selected from the group consisting of nitrogen, oxygen and sulfur. The "heterocyclyl" or "heteroalicyclyl" may be monocyclic, bicyclic, tricyclic, or tetracyclic ring system, which may be joined together in a fused, bridged or spiro-connected fashion; and the nitrogen, carbon and sulfur atoms in the "heterocyclyl" or "heteroalicyclyl" may be optionally oxidized; the nitrogen may be optionally quaternized; and the rings may also contain one or more double bonds provided that they do not form a fully delocalized pi-electron system throughout all the rings. Heterocyclyl and heteroalicyclyl groups may be unsubstituted or substituted. When substituted, the substituent(s) may be one or more groups independently selected from the group consisting of alkyl, alkenyl, alkynyl, cycloalkyl, cycloalkenyl, cycloalkynyl, aryl, heteroaryl, heteroalicyclyl, aralkyl, heteroaralkyl, (heteroalicyclyl)alkyl, hydroxy, protected hydroxyl, alkoxy, aryloxy, acyl, ester, mercapto, alkylthio, arylthio, cyano, halogen, carbonyl, thiocarbonyl, O-carbamyl, N-carbamyl, O-thiocarbamyl, N-thiocarbamyl, C-amido, N-amido, S-sulfonamido, N-sulfonamido, C-carboxy, protected C-carboxy, O-carboxy, isocyanato, thiocyanato, isothiocyanato, nitro, silyl, haloalkyl (mono-, di- and tri-substituted haloalkyl), haloalkoxy (mono-, di- and tri-substituted haloalkoxy), trihalomethanesulfonyl, trihalomethanesulfonamido, and amino, including mono- and di-substituted amino groups, and the protected derivatives thereof. Examples of such "heterocyclyl" or "heteroalicyclyl" include but are not limited to, azepinyl, acridinyl, carbazolyl, cinnolinyl, 1,3-dioxin, 1,3-dioxane, 1,4-dioxane, 1,2-dioxolanyl, 1,3-dioxolanyl, 1,4-dioxolanyl, 1,3-oxathiane, 1,4-oxathiin, 1,3-oxathiolane, 1,3-dithiole, 1,3-dithiolane, 1,4-oxathiane, tetrahydro-1,4-thiazine, 2H-1,2-oxazine, maleimide, succinimide, barbituric acid, thiobarbituric acid, dioxopiperazine, hydantoin, dihydrouracil, trioxane, hexahydro-1,3,5-triazine, imidazolinyl, imidazolidine, isoxazoline, isoxazolidine, oxazoline, oxazolidine, oxazolidinone, thiazoline, thiazolidine, morpholinyl, oxiranyl, piperidinyl *N*-Oxide, piperidinyl, piperazinyl, pyrrolidinyl, pyrrolidone, pyrrolidione, 4-piperidonyl, pyrazoline, pyrazolidinyl, 2-oxopyrrolidinyl, tetrahydropyran, 4H-pyran, tetrahydrothiopyran, thiamorpholinyl, thiamorpholinyl sulfoxide, thiamorpholinyl sulfone, and their benzo-fused analogs (e.g., benzimidazolidinone, tetrahydroquinoline, 3,4-methylenedioxyphenyl).

The term "ester" is used herein in its ordinary sense, and thus includes a chemical moiety with formula -(R)ₙ-C(=O)OR', where R and R' are independently selected from the group consisting of alkyl, cycloalkyl, aryl, heteroaryl (bonded through a ring carbon) and heteroalicyclic (bonded through a ring carbon or heteroatom), and where n is 0 or 1.

The term "amide" is used herein in its ordinary sense, and thus includes a chemical moiety with formula -(R)ₙ-C(=O)NHR' or -(R)ₙ-NHC(=O)R', where R and R' are independently selected from the group consisting of alkyl, cycloalkyl, aryl, heteroaryl (bonded through a ring carbon) and heteroalicyclic (bonded through a ring carbon or heteroatom), and where n is 0 or 1. An amide may be included in an amino acid or a peptide molecule attached to a drug molecule as described herein, thereby forming a prodrug.

Any amine, hydroxy, or carboxyl side chain on the compounds disclosed herein can be esterified or amidified. The procedures and specific groups to be used to achieve this end are known to those of skill in the art and can readily be found in reference sources such as Greene and Wuts, Protective Groups in Organic Synthesis, 3rd Ed., John Wiley & Sons, New York, NY, 1999, which is incorporated herein in its entirety.

Whenever a group is described as being "optionally substituted" that group may be unsubstituted or substituted with one or more of the indicated substituents. Likewise, when a group is described as being "unsubstituted or substituted" if substituted, the substituent may be selected from one or more the indicated substituents.

Unless otherwise indicated, when a substituent is deemed to be "optionally substituted," or "substituted" it is meant that the substituent is a group that may be substituted with one or more group(s) individually and independently selected from alkyl, alkenyl, alkynyl, cycloalkyl, cycloalkenyl, cycloalkynyl, aryl, heteroaryl, heteroalicyclyl, aralkyl, heteroaralkyl, (heteroalicyclyl)alkyl, hydroxy, protected hydroxy, alkoxy, aryloxy, acyl, ester, mercapto, cyano, halogen, carbonyl, thiocarbonyl, O-carbamyl, N-carbamyl, O-thiocarbamyl, N-thiocarbamyl, C-amido, N-amido, S-sulfonamido, N-sulfonamido, C-carboxy, protected C-carboxy, O-carboxy, isocyanato, thiocyanato, isothiocyanato, nitro, silyl, sulfenyl, sulfinyl, sulfonyl, haloalkyl (mono-, di- and tri-substituted haloalkyl), haloalkoxy (mono-, di- and tri-substituted haloalkoxy), trihalomethanesulfonyl, trihalomethanesulfonamido, and amino, including mono- and di-substituted amino groups, and the protected derivatives thereof. The protecting groups that may form the protective derivatives of the above substituents are known to those of skill in the art and may be found in references such as Greene and Wuts, Protective Groups in Organic Synthesis, 3rd Ed., John Wiley & Sons, New York, NY, 1999, which is hereby incorporated by reference in its entirety.

It is understood that, in any compound described herein having one or more chiral centers, if an absolute stereochemistry is not expressly indicated, then each center may independently be of R-configuration or S-configuration or a mixture thereof. Thus, the compounds provided herein may be enantiomerically pure or be stereoisomeric mixtures. In addition it is understood that, in any compound having one or more double bond(s) generating geometrical isomers that can be defined as E or Z each double bond may independently be E or Z or a mixture thereof. Likewise, all tautomeric forms are also intended to be included.

As used herein, the abbreviations for any protective groups, amino acids and other compounds are, unless indicated otherwise, in accord with their common usage, recognized abbreviations, or the IUPAC-IUP Commission on Biochemical Nomenclature (See, Biochem. 11:942-944 (1972)).

Embodiments disclosed herein are directed to a therapeutic composition that can include a carrier, a targeting agent operatively associated with the carrier, and a therapeutic agent operatively associated with the carrier. Various carriers can be used in the compositions disclosed herein. In some embodiments, the carrier may be selected from a non-cationic polymeric carrier, a liposome carrier, a dendritic carrier, a nanomaterial carrier, a biostructural carrier, and a micelle carrier.

### Non-Cationic Polymeric Carrier

In some embodiments, the carrier may be a non-cationic polymeric carrier. Various non-cationic polymeric carriers can be used in the compositions disclosed herein. Suitable non-cationic polymers are known to those skilled in the art. In some embodiments, the non-cationic polymeric carrier may be anionic (i.e., negatively-charged). In other embodiments, the non-cationic polymeric carrier may be electronically neutral. In some embodiments, the non-cationic polymeric carrier can be linear; in other embodiments, it can be branched. In some embodiments, the non-cationic polymeric carrier may be water-soluble. In other embodiments, the non-cationic polymeric carrier may be water-insoluble. The non-cationic polymeric carrier can be biodegradable in some embodiments. In other embodiments, the non-cationic polymeric carrier can be non-biodegradable. In some embodiments, the non-cationic polymeric carrier can include a homopolymer. In an embodiment, the non-cationic polymeric carrier may be poly-L-glutamic acid (PGA). In another embodiment, the non-cationic polymeric carrier may be poly-(y-L-glutamylglutamine) (PGGA). In still another embodiment, the non-cationic polymeric carrier may be poly-(γ-L-aspartylglutamine) (PGAA). In an embodiment, the non-cationic polymeric carrier may be a copolymer. An exemplary copolymer is poly-(lactic acid-co-glycolic acid) (PLGA). In yet other embodiments, the non-cationic polymeric carrier may include a mixture of at least two polymers.

In some embodiments, the non-cationic polymeric carrier may be in the form of a microparticle. In other embodiments, the non-cationic polymeric carrier may be in the form of a nanoparticle.

The non-cationic polymeric carrier may include a variety of recurring units. In an embodiment, the non-cationic polymeric carrier can include a recurring unit of the formula (I): 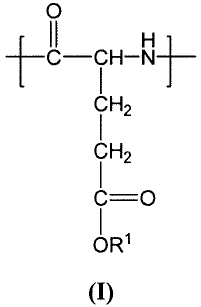 wherein the R¹ group can be hydrogen, ammonium, or an alkali metal. When the R¹ group is hydrogen, then the recurring unit of the formula (I) is a recurring unit of glutamic acid.

In other embodiments, the non-cationic polymeric carrier may include a recurring unit of the formula (II): 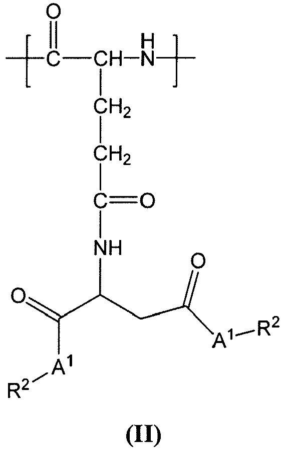 wherein each A¹ can be oxygen or NR⁴, wherein R⁴ can be hydrogen or an optionally substituted C₁₋₄ alkyl; and each R² is independently selected from hydrogen, an optionally substituted C₁₋₁₀ alkyl, an optionally substituted C₆₋₂₀ aryl, ammonium, and an alkali metal. In an embodiment, each A¹ can be oxygen and each R² can be independently selected from hydrogen and an alkali metal, e.g., sodium. When each A¹ is oxygen and each of the R² groups are hydrogen, the recurring unit of formula (II) is a recurring unit of L-aspartyl-glutamine.

In other embodiments, the non-cationic polymeric carrier may include a recurring unit of the formula (III): 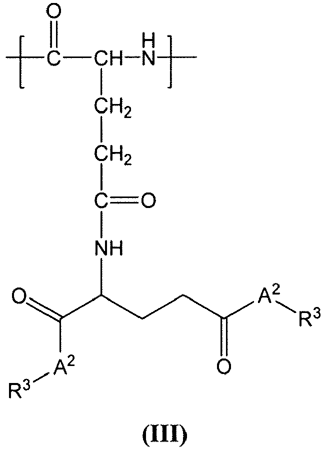 wherein each A² can be oxygen or NR⁵, wherein R⁵ can be hydrogen or an optionally substituted C₁₋₄ alkyl; and each R³ is independently selected from hydrogen, an optionally substituted C₁₋₁₀ alkyl, an optionally substituted C₆₋₂₀ aryl, ammonium, and an alkali metal. In an embodiment, each A² can be oxygen and each R³ can be independently selected from hydrogen and an alkali metal, e.g., sodium. When each A² is oxygen and each of the R³ groups are hydrogen, the recurring unit of formula (III) is a recurring unit of L-glutamyl-glutamine. Examples of alkali metals include lithium (Li), sodium (Na), potassium (K), rubidium (Rb), and cesium (Cs). In an embodiment, the alkali metal in the recurring unit(s) of formulae (II) and/or (III) can be sodium.

An embodiment provides a non-cationic polymeric carrier which can include a recurring unit of formula (IV):

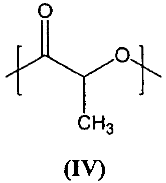

Another embodiment provides a non-cationic polymeric carrier which can include a recurring unit of formula (V):

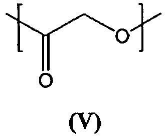

In an embodiment, the non-cationic polymeric carrier includes a recurring unit selected from formula (IV) and formula (V). In another embodiment, the non-cationic polymeric carrier may include a recurring unit of the formula (IV) and a recurring unit of the formula (V). When the non-cationic polymeric carrier includes both a recurring unit of formula (IV) and a recurring unit of formula (V), the carrier may be PLGA.

As mentioned previously, the non-cationic polymeric carrier can be a homopolymer. For example, the non-cationic polymeric carrier may consist entirely of recurring units of formula (I). Alternatively, the non-cationic polymeric carrier may consist entirely of recurring units of formula (II). In an embodiment, the non-cationic polymeric carrier may consist entirely of recurring units of formula (III). In other embodiments, the non-cationic polymeric carrier may consist entirely of recurring units of formula (IV) or formula (V).

However, the non-cationic polymeric carrier may be a copolymer. When the non-cationic polymeric carrier is a copolymer, the copolymer may include one, two or more recurring units of the formulae (I), (II), (III), (IV), and (V). In some embodiments, the non-cationic polymeric carrier can be a copolymer that includes at least two different recurring units selected from the formulae (I), (II), (III), (IV), and (V). In some embodiments, the non-cationic polymeric carrier can be a copolymer that includes other recurring units, e.g., recurring units formed by copolymerization methods using monomers that are readily copolymerizable with the monomers used to form the recurring units of the formulae (I), (II), (III), (IV), and (V). Routine experimentation guided by the disclosure provided herein may be used to identify such comonomers and polymerization conditions.

The non-cationic polymeric carrier can contain one or more chiral carbon atoms. The chiral carbon (which may be indicated by an asterisk *) can have the *rectus* (right handed) or the *sinister* (left handed) configuration, and thus the recurring unit may be racemic, enantiomeric or enantiomerically enriched.

The percentage of the recurring unit of the formula (I) and/or the formula (II) in the non-cationic polymeric carrier may vary over a wide range. In an embodiment, the non-cationic polymeric carrier may include about 1 mole % to about 99 mole % of the recurring unit of formula (I) and/or the formula (II), based on the total moles of recurring units in the non-cationic polymeric carrier. In another embodiment, the non-cationic polymeric carrier may include about 1 mole % to about 50 mole % of the recurring unit of formula (I) and/or the formula (II), based on the total moles of recurring units in the non-cationic polymeric carrier. In another embodiment, the non-cationic polymeric carrier may include about 1 mole % to about 30 mole % of the recurring unit of formula (I) and/or the formula (II), based on the total moles of recurring units in the non-cationic polymeric carrier. In another embodiment, the non-cationic polymeric carrier may include about 1 mole % to about 20 mole % of the recurring unit of formula (I) and/or the formula (II), based on the total moles of recurring units in the non-cationic polymeric carrier. In another embodiment, the non-cationic polymeric carrier may include about 1 mole % to about 10 mole % of the recurring unit of formula (I) and/or the formula (II), based on the total moles of recurring units in the non-cationic polymeric carrier.

Similarly, the percentage of the recurring units of formula (III) in the non-cationic polymeric carrier may vary over a wide range. In an embodiment, the non-cationic polymeric carrier may include about 1 mole % to about 99 mole % of the recurring unit of formula (III), based on the total moles of recurring units in the non-cationic polymeric carrier. In another embodiment, the non-cationic polymeric carrier may include about 1 mole % to about 50 mole % of the recurring unit of formula (III), based on the total moles of recurring units in the non-cationic polymeric carrier. In another embodiment, the non-cationic polymeric carrier may include about 1 mole % to about 30 mole % of the recurring unit of formula (III), based on the total moles of recurring units in the non-cationic polymeric carrier. In another embodiment, the non-cationic polymeric carrier may include about 1 mole % to about 20 mole % of the recurring unit of formula (III), based on the total moles of recurring units in the non-cationic polymeric carrier. In another embodiment, the non-cationic polymeric carrier may include about 1 mole % to about 10 mole % of the recurring unit of formula (III), based on the total moles of recurring units in the non-cationic polymeric carrier.

In an embodiment, the non-cationic polymeric carrier can be a copolymer including a recurring unit of the formula (II) and a recurring unit of the formula (III). Non-cationic polymeric carriers that include a recurring unit of the formula (II) and a recurring unit of the formula (III) may be copolymers that include other recurring units that are not of the formula (II) and not of the formula (III). Exemplary other recurring units not of formula (II) or formula (III), include but are not limited to, recurring units of the formulae (I), (IV) and (V).

The percentage of recurring units of formula (II), based on the total number of recurring units in the non-cationic polymeric carrier comprising recurring units of formulae (II), and (III), may vary over a wide range. In an embodiment, the non-cationic polymeric carrier may comprise about 1 mole % to about 99 mole % of the recurring unit of formula (II) based on the total moles of recurring units of formulae (II) and (III). In another embodiment, the non-cationic polymeric carrier may comprise about 1 mole % to about 50 mole % of the recurring unit of formula (II) based on the total moles of recurring units of formulae (II) and (III). In another embodiment, the non-cationic polymeric carrier may comprise about 1 mole % to about 30 mole % of the recurring unit of formula (II) based on the total moles of recurring units of formulae (II) and (III). In another embodiment, the non-cationic polymeric carrier may comprise about 1 mole % to about 20 mole % of the recurring unit of formula (II) based on the total moles of recurring units of formulae (II) and (III). In another embodiment, the non-cationic polymeric carrier may comprise about 1 mole % to about 10 mole % of the recurring unit of formula (II) based on the total moles of recurring units of formulae (II) and (III).

With respect to the recurring unit of formula (III), in an embodiment, the non-cationic polymeric carrier may comprise about 1 mole % to about 99 mole % of the recurring unit of formula (III) based on the total moles of recurring units of formulae (II) and (III). In another embodiment, the non-cationic polymeric carrier may comprise about 1 mole % to about 50 mole % of the recurring unit of formula (III) based on the total moles of recurring units of formulae (II) and (III). In another embodiment, the non-cationic polymeric carrier may comprise about 1 mole % to about 30 mole % of the recurring unit of formula (III) based on the total moles of recurring units of formulae (II) and (III). In another embodiment, the non-cationic polymeric carrier may comprise about 1 mole % to about 20 mole % of the recurring unit of formula (III) based on the total moles of recurring units of formulae (II) and (III). In another embodiment, the non-cationic polymeric carrier may comprise about 1 mole % to about 10 mole % of the recurring unit of formula (III) based on the total moles of recurring units of formulae (II) and (III).

In an embodiment, the non-cationic polymeric carrier may include a recurring unit of formula (I), a recurring unit of formula (II), and a recurring unit of formula (III). The percentage of recurring units of formula (II), based on the total number of recurring units in the non-cationic polymeric carrier comprising recurring units of formulae (I), (II), and (III), may vary over a wide range. In an embodiment, the non-cationic polymeric carrier may comprise about 1 mole % to about 99 mole % of the recurring unit of formula (II) based on the total moles of recurring units of formulae (I), (II) and (III). In another embodiment, the non-cationic polymeric carrier may comprise about 1 mole % to about 50 mole % of the recurring unit of formula (II) based on the total moles of recurring units of formulae (I), (II) and (III). In another embodiment, the non-cationic polymeric carrier may comprise about 1 mole % to about 30 mole % of the recurring unit of formula (II) based on the total moles of recurring units of formulae (I), (II) and (III). In another embodiment, the non-cationic polymeric carrier may comprise about 1 mole % to about 20 mole % of the recurring unit of formula (II) based on the total moles of recurring units of formulae (I), (II) and (III). In another embodiment, the non-cationic polymeric carrier may comprise about 1 mole % to about 10 mole % of the recurring unit of formula (II) based on the total moles of recurring units of formulae (I), (II) and (III).

With respect to the recurring unit of formula (III), in an embodiment, the non-cationic polymeric carrier may comprise about 1 mole % to about 99 mole % of the recurring unit of formula (III) based on the total moles of recurring units of formulae (I), (II), and (III). In another embodiment, the non-cationic polymeric carrier may comprise about 1 mole % to about 50 mole % of the recurring unit of formula (III) based on the total moles of recurring units of formulae (I), (II), and (III). In another embodiment, the non-cationic polymeric carrier may comprise about 1 mole % to about 30 mole % of the recurring unit of formula (III) based on the total moles of recurring units of formulae (I), (II), and (III). In another embodiment, the non-cationic polymeric carrier may comprise about 1 mole % to about 20 mole % of the recurring unit of formula (III) based on the total moles of recurring units of formulae (I), (II), and (III). In another embodiment, the non-cationic polymeric carrier may comprise about 1 mole % to about 10 mole % of the recurring unit of formula (III) based on the total moles of recurring units of formulae (I), (II), and (III).

As with recurring units of formulae (II) and (III), the percentage of recurring units of formula (I) may vary over a wide range. In an embodiment, the non-cationic polymeric carrier may comprise about 1 mole % to about 99 mole % of the recurring unit of formula (I) based on the total moles of recurring units of formulae (I), (II) and (III). In another embodiment, the non-cationic polymeric carrier may comprise about 1 mole % to about 50 mole % of the recurring unit of formula (I) based on the total moles of recurring units of formulae (I), (II) and (III). In another embodiment, the non-cationic polymeric carrier may comprise about 1 mole % to about 30 mole % of the recurring unit of formula (I) based on the total moles of recurring units of formulae (I), (II) and (III). In another embodiment, the non-cationic polymeric carrier may comprise about 1 mole % to about 20 mole % of the recurring unit of formula (I) based on the total moles of recurring units of formulae (I), (II) and (III). In another embodiment, the non-cationic polymeric carrier may comprise about 1 mole % to about 10 mole % of the recurring unit of formula (I) based on the total moles of recurring units of formulae (I), (II) and (III).

In some embodiments, the non-cationic polymeric carrier can be a copolymer that includes a recurring unit of formula (IV). In an embodiment, the non-cationic polymeric carrier can be a copolymer that includes a recurring unit of formula (V). In an embodiment, the non-cationic polymeric carrier can be a copolymer that includes a recurring unit of formula (IV) and a recurring unit of formula (V).

The percentage of the recurring unit of the formula (IV) in the non-cationic polymeric carrier may vary over a wide range. In an embodiment, the non-cationic polymeric carrier may include about 1 mole % to about 99 mole % of the recurring unit of formula (IV), based on the total moles of recurring units in the non-cationic polymeric carrier. In another embodiment, the non-cationic polymeric carrier may include about 1 mole % to about 50 mole % of the recurring unit of formula (IV), based on the total moles of recurring units in the non-cationic polymeric carrier. In another embodiment, the non-cationic polymeric carrier may include about 1 mole % to about 30 mole % of the recurring unit of formula (IV), based on the total moles of recurring units in the non-cationic polymeric carrier. In another embodiment, the non-cationic polymeric carrier may include about 1 mole % to about 20 mole % of the recurring unit of formula (IV), based on the total moles of recurring units in the non-cationic polymeric carrier. In another embodiment, the non-cationic polymeric carrier may include about 1 mole % to about 10 mole % of the recurring unit of formula (IV), based on the total moles of recurring units in the non-cationic polymeric carrier.

Likewise, the percentage of the recurring unit of the formula (V) in the non-cationic polymeric carrier may vary over a wide range. In an embodiment, the non-cationic polymeric carrier may include about 1 mole % to about 99 mole % of the recurring unit of formula (V), based on the total moles of recurring units in the non-cationic polymeric carrier. In another embodiment, the non-cationic polymeric carrier may include about 1 mole % to about 50 mole % of the recurring unit of formula (V), based on the total moles of recurring units in the non-cationic polymeric carrier. In another embodiment, the non-cationic polymeric carrier may include about 1 mole % to about 30 mole % of the recurring unit of formula (V), based on the total moles of recurring units in the non-cationic polymeric carrier. In another embodiment, the non-cationic polymeric carrier may include about 1 mole % to about 20 mole % of the recurring unit of formula (V), based on the total moles of recurring units in the non-cationic polymeric carrier. In another embodiment, the non-cationic polymeric carrier may include about 1 mole % to about 10 mole % of the recurring unit of formula (V), based on the total moles of recurring units in the non-cationic polymeric carrier.

In an embodiment, the non-cationic polymeric carrier may include about 1 mole % to about 99 mole % of the recurring unit of formula (IV), based on the total moles of recurring units of formulae (IV) and (V). In another embodiment, the non-cationic polymeric carrier may include about 1 mole % to about 50 mole % of the recurring unit of formula (IV), based on the total moles of recurring units of formulae (IV) and (V). In another embodiment, the non-cationic polymeric carrier may include about 1 mole % to about 30 mole % of the recurring unit of formula (IV), based on the total moles of recurring units of formulae (IV) and (V). In another embodiment, the non-cationic polymeric carrier may include about 1 mole % to about 20 mole % of the recurring unit of formula (IV), based on the total moles of recurring units of formulae (IV) and (V). In another embodiment, the non-cationic polymeric carrier may include about 1 mole % to about 10 mole % of the recurring unit of formula (IV), based on the total moles of recurring units of formulae (IV) and (V).

In an embodiment, the non-cationic polymeric carrier may include about 1 mole % to about 99 mole % of the recurring unit of formula (V), based on the total moles of recurring units of formulae (IV) and (V). In another embodiment, the non-cationic polymeric carrier may include about 1 mole % to about 50 mole % of the recurring unit of formula (V), based on the total moles of recurring units of formulae (IV) and (V). In another embodiment, the non-cationic polymeric carrier may include about 1 mole % to about 30 mole % of the recurring unit of formula (V), based on the total moles of recurring units of formulae (IV) and (V). In another embodiment, the non-cationic polymeric carrier may include about 1 mole % to about 20 mole % of the recurring unit of formula (V), based on the total moles of recurring units of formulae (IV) and (V). In another embodiment, the non-cationic polymeric carrier may include about 1 mole % to about 10 mole % of the recurring unit of formula (V), based on the total moles of recurring units of formulae (IV) and (V).

The non-cationic polymeric carriers disclosed herein may be commercially available and/or produced according to methods known to those skilled in the art. In an embodiment, a non-cationic polymeric carrier that includes a recurring unit of the formula (II) can be produced starting with polyglutamic acid and an amino acid such as aspartic acid. Alternatively, in another embodiment, the non-cationic polymeric carrier may be created by first converting the starting polyglutamic acid material into its salt form. The salt form of polyglutamic can be obtained by reacting polyglutamic acid with a suitable base, e.g., sodium bicarbonate. An aspartic acid moiety can be attached to the pendant carboxylic acid or carboxylate group of the polyglutamic acid. The weight average molecular weight of the polyglutamic acid is not limited, but is preferably from about 10,000 to about 500,000 daltons, and more preferably from about 25,000 to about 300,000 daltons. A non-cationic polymeric carrier that includes a recurring unit of formula (III) can be made following the same or similar procedure using glutamic acid in place of aspartic acid. The aforementioned reactions may be used to create poly-(γ-L-aspartyl-glutamine) or poly-(y-L-glutamylglutamine). Further details regarding some of the non-cationic polymeric carriers mentioned above and their synthesis can be found in U.S. Patent Publication No. 2007-0128118, titled "POLYGLUTAMATE-AMINO ACID CONJUGATES AND METHODS," filed on December 1, 2006 and incorporated herein by reference in its entirety.

A non-cationic polymeric carrier that includes a recurring unit of the formula (IV) may be commercially available and can be produced according methods known to those of skill in the art. In an embodiment, a non-cationic polymeric carrier that includes a recurring unit of the formula (IV) may be produced starting with lactic acid. Lactic acid may be reacted to obtain lactide, which may then be polymerized. A non-cationic polymeric carrier that includes a recurring unit of the formula (V) may also be commercially available and can be produced according to methods known to those of skill in the art. In an embodiment, a non-cationic polymeric carrier that includes a recurring unit of the formula (V) may be produced by reacting chloroacetic acid with a suitable base, e.g., sodium hydroxide. As previously mentioned, the non-cationic polymeric carrier can be a copolymer that includes a recurring unit of formula (IV) and a recurring unit of formula (V). When the non-cationic polymeric carrier includes both a recurring unit of formula (IV) and a recurring unit of formula (V), the carrier may be PLGA. Such a copolymer may be commercially available and can be prepared according to methods known to those of skill in the art.

Those of ordinary skill will appreciate that in order to make the carrier non-cationic, the salt form of the starting monomer may be used. Likewise, after polymerization, the resulting polymer can be treated with a base to neutralize any residual positive charge.

### Liposome Carrier

In some embodiments, the carrier may be a liposome carrier. Various liposome carriers can be used in the compositions disclosed herein. Suitable liposome carriers are known to those skilled in the art, and can be selected on the basis of various properties, such as rigidity of the lipid bilayer, the electronic charge of the lipid bilayer and/or the compatibility of the liposome carrier with one or both of the agents. In some embodiments, the liposome carrier can include a phospholipid. In an embodiment, the liposome carrier can include a natural phospholipid, such as egg phosphatidylcholine, egg phosphatidylethanolamine, soy bean phosphatidylcholine, lecithin, and sphingomyelin. In other embodiments, the liposome carrier can include a synthetic phospholipid. Synthetic phospholipids include, but are not limited to, synthetic phosphatidylcholine, lyso-phosphatidylcholine, phosphatidylglycerol, phosphatidic acid, phosphatidylethanolamine, phosphatidylserine, and derivatives thereof. Synthetic phospholipids may be derivatized in various ways, e.g., a synthetic phospholipid may be a so-called "PEGylated" phospholipid that comprises one or more polyethylene glycol moieties.

In some embodiments, the liposome carrier may be cationic. In other embodiments, the liposome carrier may be electronically neutral. In still other embodiments, the liposome carrier may be anionic. Those skilled in the art will recognize that various lipids may be selected to obtain a desired net electronic charge for the liposome carrier.

### Dendritic Carrier

In some embodiments, the carrier may be a dendritic carrier. Various dendritic carriers can be used in the compositions disclosed herein. Suitable dendritic carriers are known to those skilled in the art, and can be selected depending on the agents with which it is operatively associated as well as the desired properties of the dendritic carrier. In some embodiments, the dendritic carrier can be a dendrimer. In an embodiment, the dendritic carrier can be a dendron.

As discussed previously, a dendritic carrier includes a core molecule. Exemplary core molecules include, but are not limited to, an alkyl diamine such as ethylenediamine, 1,4-diaminobutane, 1,6-diaminohexane, and 1,12-diaminodecane; an amine such as ammonia; cystamine; an alkyl imine such as poly(ethyleneimine) (PEI); and a chlorinated phosphorus molecule such as cyclotriphosphazene and thiophosphoryl. A dendritic carrier typically includes one or more branching groups. Exemplary branching groups include, but are not limited to, an alkyl imine such as poly(propyleneimine) (PPI) (e.g., DAB-Am-16), a tertiary amine such as a poly(amidoamine) (PAMAM), a poly(amino acid) such as poly(lysine), and phenoxymethyl(methylhydrazono) (PMMH).

In some embodiments, the branching groups cannot include poly(propyleneimine). In other embodiments, the branching groups cannot include poly(amidoamine). In still other embodiments, the branching groups cannot include poly(lysine). In some embodiments, the dendritic carrier cannot include poly(propyleneimine). In other embodiments, the dendritic carrier cannot include poly(amidoamine). In still other embodiments, the dendritic carrier cannot include lysine and/or poly(lysine). In an embodiment, the dendritic carrier cannot be PAMAM. In an embodiment, the dendritic carrier cannot be DAB-Am-16. In an embodiment, the dendritic carrier cannot be poly(lysine).

The number of branches of the dendritic carrier can vary. In an embodiment, the dendritic carrier can include two or more branches. In another embodiment, the dendritic carrier can include three or more branches. In still other embodiments, the dendritic carrier can include four or more branches. Furthermore, as discussed previously, the dendritic carrier can include one or more generations. Each generation of branching groups may be synthesized through an iterative or repeated chemical reaction. In some embodiments, the dendritic carrier can include one generation of branching groups. In other embodiments, the dendritic carrier can include two generations of branching groups. In still other embodiments, the dendritic carrier can include two or more generations of branching groups.

In some embodiments, at least a portion of the dendritic carrier may be hydrophobic. In some embodiments, at least a portion of the dendritic carrier may be hydrophilic. In an embodiment, a portion of the dendritic carrier may be hydrophobic, and a different portion of the dendritic carrier may be hydrophilic. In some embodiments, the dendritic carrier may be cationic. In other embodiments, the dendritic carrier may be electronically neutral. In still other embodiments, the dendritic carrier may be anionic. Those skilled in the art will recognize that various starting materials may be selected to obtain a dendritic carrier that exhibits the desired properties.

### Nanomaterial Carrier

In some embodiments, the carrier may be a nanomaterial carrier. Various nanomaterial carriers can be used in the compositions disclosed herein. Suitable nanomaterial carriers are known to those skilled in the art, and can be selected on a basis informed by the guidance provided herein, depending on the agents with which it is operatively associated. In some embodiments, the nanomaterial carrier may be a nanoparticle. In some embodiments, the nanoparticle may include a metal. In an embodiment, the nanoparticle may be a gold nanoparticle. In an embodiment, the gold nanoparticle can be positively charged. In another embodiment, the gold nanoparticle can be negatively charged. In some embodiments, the nanoparticle may be a nanosphere.

In an embodiment, the nanoparticle may be a nanopolymer. Examples of polymers that can be nanopolymers include, but are not limited to, poly-(lactic-co-glycolic acid) (PLGA), polyalkylcyanoacrylate (PACA), polyepsilon-caprolactone (PCL), and polylactide (PLA). In an embodiment, the nanomaterial carrier includes PLGA and polyethylene glycol (PEG).

In other embodiments, the nanoparticle may be a fullerene. A fullerene may exist in a variety of shapes, including but not limited to, spherical, ellipsoid, cylindrical, and planar. In some embodiments, the cylindrically-shaped fullerene nanoparticle may be a carbon nanotube. In an embodiment, the carbon nanotube can be single-walled. In another embodiment, the carbon nanotube can be multi-walled. The carbon nanotube may be of various orientations, including, but not limited to, armchair, zig-zag, and chiral. The diameter of the carbon nanotube can be in the range of about 0.1 nm to about 10 nm. An exemplary diameter is approximately 1 nm. In an embodiment, an additional functional group may be attached to the carbon nanotube. In some embodiments, an additional functional group may be attached to the carbon nanotube to promote transport across a cell membrane. For example, one or more hydrophilic groups may be attached to a terminus of the carbon nanotube.

### Biostructural Carrier

In some embodiments, the carrier may be a biostructural carrier. Various biostructural carriers can be used in the compositions disclosed herein. Suitable biostructural carriers are known to those skilled in the art, and can be selected on a basis informed by the guidance provided herein, depending on the agents with which it is operatively associated.

In some embodiments, the majority of units in the biostructural carrier can be saccharide units. One such biostructural carrier in which the majority of units are saccharides is a sugar. In an embodiment, the biostructural carrier can be a monosaccharide or a semi-synthetic derivative thereof. In another embodiment, the biostructural carrier can be an oligosaccharide or a semi-synthetic derivative thereof. In some embodiments, the biostructural carrier can include a polysaccharide or a semi-synthetic derivative thereof, such as a cyclic polysaccharide, a linear polysaccharide, and a branched polysaccharide. An exemplary non-cyclic polysaccharide is dextran. In some embodiments, dextran can have a molecular weight in the range of about 1 kilodalton (kDa) to about 2,000 kDa. In other embodiments, the molecular weight can range from about 1 kDa to about 500 kDa. In still other embodiments, the molecular weight can range from about 10 kDa to about 500 kDa. In some embodiments, the molecular weight of dextran can be selected from approximately 1kDa, approximately 10 kDa, approximately 20 kDa, approximately 40 kDa, approximately 60 kDa, approximately 70 kDa, and approximately 500 kDa.

An exemplary cyclic polysaccharide is a cyclodextrin. Exemplary cyclodextrins include, but are not limited to, α-cyclodextrin, β-cyclodextrin, and γ-cyclodextrin. The cyclodextrin can be unsubstituted or substituted. In some embodiments, one or more pendant hydroxyl groups may be substituted for another substituent. Exemplary substituents include, but are not limited to, an alkyl, dialkyl, carboxyalkyl, hydroxyalkyl, alkoxy, sulfoalkyl, and/or glucose group. Exemplary substituted cyclodextrin biostructural carriers include, but are not limited to, methyl β-cyclodextrin, dimethyl-β-cyclodextrin, carboxymethyl-β-cyclodextrin, hydroxypropyl β-cyclodextrin, sulfobutylether-β-cyclodextrin, tri-O-methyl-β-cyclodextrin, and glucosyl-β-cyclodextrin. Those skilled in the art will appreciate that the choice of a particular substituent as well as the degree of substitution can vary according to the desired properties of the biostructural carrier, e.g., polarity and/or hydrophobicity.

The number of saccharide units present in a biostructural carrier can vary. In some embodiments, the biostructural carrier can contain one or more saccharide units. In other embodiments, the biostructural carrier can contain five or more saccharide units. In still other embodiments, the biostructural carrier can contain seven or more saccharide units. In yet still other embodiments, the biostructural carrier can contain ten or more saccharide units. In an embodiment, the biostructural carrier can have been five to fifty saccharide units. In another embodiment, the biostructural carrier can have been five to twenty saccharide units. In yet another embodiment, the biostructural carrier can have been five to fifteen saccharide units.

In some embodiments, the majority of units in the biostructural carrier can be amino acid units. In some embodiments, the amino acid units are not lysine, such as L-lysine. Examples of the biostructural carrier in which the majority of units are amino acids are proteins and peptides. In some embodiments, the biostructural carrier may include albumin. Exemplary types of albumin include, but are not limited to, C-reactive protein, conalbumin, lactalbumin, ovalbumin, parvalbumin, serum albumin, and technetium TC 99m aggregated albumin. Exemplary types of serum albumin include, but are not limited to, human serum albumin (HSA) and bovine serum albumin (BSA). In some embodiments, the protein or peptide may be naturally-derived; in other embodiments, the protein or peptide may be synthetic (e.g., recombinant human serum albumin (rHSA)). In some embodiments, the biostructural carrier does not include poly-L-lysine. In an embodiment, the biostructural carrier cannot be poly-L-lysine.

The number of amino acid units present in a biostructural carrier can vary. In some embodiments, the biostructural carrier can include 2 to 1500 amino acid units. In other embodiments, the biostructural carrier can include 2 to 50 amino acid units. In still other embodiments, the biostructural carrier can include 8 to 35 amino acid units. In yet still other embodiments, the biostructural carrier can include 15 to 30 amino acid units. In an embodiment, the biostructural carrier can include 80 to 1250 amino acid units. In another embodiment, the biostructural carrier can include 100 to 1000 amino acid units. In still another embodiment, the biostructural carrier can include 200 to 700 amino acid units. In some embodiments, at least a portion of the biostructural carrier may be hydrophobic. In some embodiments, at least a portion of the biostructural carrier may be hydrophilic. In an embodiment, a portion of the biostructural carrier may be hydrophobic, and a different portion of the biostructural carrier may be hydrophilic. In some embodiments, the biostructural carrier may be cationic. In other embodiments, the biostructural carrier may be electronically neutral. In still other embodiments, the biostructural carrier may be anionic. Those skilled in the art will recognize that various starting materials may be selected to obtain a biostructural carrier that exhibits the desired properties, as informed by the guidance provided herein.

Various molecular weights of the biostructural carrier can be used in the compositions described herein. When the biostructural carrier includes a majority of saccharide units, the molecular weight can range from about 500 Daltons to about 2,500 Daltons. In some embodiments, the molecular weight can range from about 1,000 Daltons to about 2,000 Daltons. In still other embodiments, the molecular weight can range from about 1,000 Daltons to about 1,500 Daltons. When the biostructural carrier includes a majority of amino acid units, the molecular weight can range from about 20,000 Daltons to about 100,000 Daltons. In some embodiments, the molecular weight can range from about 30,000 Daltons to about 70,000 Daltons. In other embodiments, the molecular weight can range from about 50,000 Daltons to about 100,000 Daltons. Those skilled in the art will appreciate that, in determining the molecular weight of the biostructural carrier, the molecular weight of the agent(s) is not taken into account.

### Micelle Carrier

In some embodiments, the carrier may be a micelle carrier. Various micelle carriers can be used in the compositions disclosed herein. Suitable micelle carriers are known to those skilled in the art, and can be selected depending on the agents with which it is operatively associated. In some embodiments, the micelle carrier can include a lipid. Exemplary lipids include, but are not limited to, a fatty acid and a phospholipid. In other embodiments, the micelle carrier may include a polymer. In an embodiment, the micelle carrier can include a homopolymer. Exemplary homopolymers include, but are not limited to, a poly(alkylene glycol) such as poly(ethylene glycol) (PEG), a poly(amino acid) such as poly(aspartic acid) and poly(glutamic acid) (PGA), poly-(γ-L-glutamylglutamine) (PGGA), poly(phenylene oxide) (PPO), poly(ε-caprolactone) (PCL), and poly(lactic acid). In an embodiment, the micelle carrier may include poly-(γ-L-glutamylglutamine) (PGGA). In other embodiments, the micelle carrier can include a copolymer, such as poly (lactic-co-glycolic acid) (PLGA). In some embodiments, the micelle carrier can include a block copolymer. An exemplary block copolymer is a diblock copolymer. In some embodiments, the diblock copolymer can include a nonpolar recurring unit and a polar recurring unit. Exemplary polar recurring units include, but are not limited to, an alkylene glycol such as ethylene glycol, an alkylene oxide such as ethylene oxide, and a hydrophilic amino acid. Exemplary nonpolar recurring units include, but are not limited to, γ-L-glutamylglutamine, glutamic acid, lactic-co-glycolic acid, phenylene oxide, ε-caprolactone, lactic acid, styrene, butylene oxide, a hydrocarbon, and a hydrophobic amino acid such as aspartic acid. Other block copolymers with more than two different recurring units may be used, such as a triblock copolymer. In other embodiments, the micelle carrier cannot include a polymer. In an embodiment, the micelle carrier is a non-polymeric micelle carrier.

In some embodiments, the micelle carrier may be cationic. In other embodiments, the micelle carrier may be electronically neutral. In still other embodiments, the micelle carrier may be anionic. Those skilled in the art will recognize that various starting materials may be selected to obtain a desired net electronic charge for the micelle carrier.

As with the carrier, various targeting agents may be used in a therapeutic composition. In the therapeutic composition of the present invention, the targeting agent includes a retinoid, such as those described herein. Suitable retinoids include retinol, retinal, retinoic acid, rexinoid, or derivatives or analogs thereof. Exemplary retinols include vitamin A, all-trans retinol, retinyl palmitate, and retinyl acetate. One example of a retinal is 11-cis-retinal. Rexinoids are retinoid compounds which are selective for retinoid X receptors (RXR). An exemplary rexinoid is bexarotene. Other retinoid derivatives and analogs include etretinate, acitretin, tazarotene, bexarotene, adapalene, and fenretinide. In some embodiments, the retinoid can be selected from retinol, retinal, retinoic acid, all-trans retinol, all-trans retinoic acid, retinyl palmitate, 11-cis-retinal and 13-cis-retinoic acid. In an embodiment, the retinoid may include vitamin A.

As mentioned previously, the targeting agent may increase the delivery selectivity of the therapeutic composition to a particular target organ or tissue. Target organs may include, for example, the liver, pancreas, kidney, lung, esophagus, larynx, bone marrow, and brain. In some embodiments, the increase in delivery selectivity may be at least about two-fold as compared to that of an otherwise comparable therapeutic composition lacking the targeting agent. In an embodiment, the increase in delivery selectivity may be at least threefold. In some embodiments, the therapeutic compositions described herein can increase the delivery of the therapeutic agent to the target organ by at least 10% more as compared to that of an otherwise comparable therapeutic composition lacking the target agent. In other embodiments, the therapeutic compositions described herein can increase the delivery of the therapeutic agent to the target organ by at least 25% more as compared to that of an otherwise comparable therapeutic composition lacking the target agent. In yet other embodiments, the therapeutic compositions described herein can increase the delivery of the therapeutic agent to the target organ by at least 50% more as compared to that of an otherwise comparable therapeutic composition lacking the target agent. In yet still other embodiments, the therapeutic compositions described herein can increase the delivery of the therapeutic agent to the target organ by at least 75% more as compared to that of an otherwise comparable therapeutic composition lacking the target agent.

The amount of targeting agent present in the therapeutic composition can vary over a wide range. In some embodiments, the targeting agent can be about 1% to about 50% (weight/weight) of the total mass of the therapeutic composition (wherein the mass of the targeting agent is included in the total mass of the therapeutic composition). In other embodiments, the targeting agent may be about 10% to about 30% w/w of the total mass of the therapeutic composition (same basis). In still other embodiments, the targeting agent may be about 20% to about 40% w/w of the total mass of the therapeutic composition (same basis).

A variety of therapeutic agents may be included in the therapeutic compositions described herein. In some embodiments, the therapeutic activity of the therapeutic agent may be inhibiting the growth of a cancer cell. The therapeutic agent may directly and/or indirectly inhibit the growth of a cancer cell. For example, the therapeutic agent may induce apoptosis by directly acting on the cancer cell. The therapeutic agent may also indirectly inhibit the growth of a cancer cell by targeting one or more fibroblast cells that supports the cancer cell. In an embodiment, the therapeutic agent may be cytotoxic.

In some embodiments, the therapeutic activity of the therapeutic agent may include inhibiting fibrosis within a target organ or tissue, such as those described previously. For example, the therapeutic agent may inhibit the activation of a stellate cell upon delivery of the therapeutic agent to a target organ or tissue. "Activation," as the term is used herein, describes an abnormal state of a stellate cell characterized by increased proliferation, decreased vitamin A concentration, and/or increased collagen production.

In some embodiments, the therapeutic agent may be an anti-cancer agent. An exemplary anti-cancer agent is paclitaxel. In some embodiments, the therapeutic agent may be a small molecule agent. In these embodiments, the therapeutic agent may be selected from a transforming growth factor beta (TGFβ) inhibitor, a matrix metalloproteinase (MMP) promoter, a hepatocyte growth factor (HGF) promoter, a tissue inhibitor of metalloproteinase (TIMP) production inhibitor, a gamma-type peroxisome proliferator-activated receptor (PPARγ) ligand, an angiotensin activity inhibitor, a platelet derived growth factor (PDGF) inhibitor, a sodium channel inhibitor, and an apoptosis inducer.

In other embodiments, the therapeutic agent may include an amino acid. In these embodiments, the therapeutic agent may be selected from siRNA, DNA, RNA, and an antisense nucleic acid. In an embodiment, the therapeutic agent can be siRNA. In some embodiments, siRNA includes RNA having 5 to 50 base pairs, preferably 10 to 35 base pairs and more preferably 19 to 27 base pairs. The siRNA may also include mixed RNA/DNA molecules or mixed protein/RNA molecules. In an embodiment, the therapeutic agent may inhibit the secretion of collagen. The therapeutic agent may, upon delivery to a target organ, substantially inhibit the activity of a tissue inhibitor of metalloproteinases (TIMP) or a molecular chaperone. In some embodiments, the molecular chaperone that is inhibited by delivery of the therapeutic agent to a target organ may collagen-specific, such as heat shock protein 47 (HSP47).

The amount of therapeutic agent present in the therapeutic composition can vary over a wide range. The therapeutic agent can be about 25% to about 75% (weight/weight) of the total mass of the therapeutic composition (wherein the mass of the therapeutic agent is included in the total mass of the therapeutic composition). In other embodiments, the therapeutic agent can be about 30% to about 60% w/w of the total mass of the therapeutic composition (same basis). In still other embodiments, the therapeutic agent can be about 40% to about 70% w/w of the total mass of the therapeutic composition (same basis).

The therapeutic compositions disclosed herein may be prepared in various ways. One or more of the targeting agents and/or therapeutic agents disclosed herein can be operatively associated with the carrier through an electrostatic association. In an embodiment, the targeting agent may be operatively associated with the carrier through an electrostatic association. Likewise, the therapeutic agent may be operatively associated with the carrier through an electrostatic association. In some embodiments where the therapeutic agent is associated with the carrier through an electrostatic association, the therapeutic agent may include an amino acid. For example, the therapeutic agent siRNA may be electrostatically associated with the carrier.

Alternatively, in some embodiments, one or more of the agents may be operatively associated with the carrier through a covalent bond. When operatively associated through a covalent bond, one or more of the agents may be directly bonded to the carrier. A variety of mechanisms known to those skilled in the art can be used to form the covalent bond between the one or more agents and carrier, such as a condensation reaction. Additional methods for directly bonding one or more agents to a carrier are known to those skilled in the art, and may be identified by routine experimentation informed by the guidance provided herein.

In some embodiments, the targeting agent may be operatively associated with the carrier through a covalent bond. For example, when a non-cationic polymeric carrier is used, retinol may be directly bonded to one or more of the recurring units (e.g., formulae (I), (II), (III), (IV), and (V)) described herein through a condensation reaction. In some embodiments, the therapeutic agent may be operatively associated with the carrier through a covalent bond. For example, an anti-cancer agent may be directly bonded to the carrier. In an embodiment, paclitaxel can be operatively associated with the carrier at the oxygen atom attached to the C2'-carbon. In another embodiment, paclitaxel can be operatively associated with the carrier at the oxygen atom attached to the C7-carbon. In some embodiments, the carrier can have paclitaxel attached at the oxygen atom attached to the C2'-carbon and/or the oxygen atom attached to the C7-carbon.

In other embodiments, one or more of the agents described herein may be operatively associated with the carrier through a linking group. Examples of linking groups include relatively low molecular weight groups such as amide, ester, carbonate and ether, as well as higher molecular weight linking groups such as poly(ethylene glycol) (PEG). In an embodiment, the linking group may be acid labile. In some embodiments, an interior and/or exterior moiety or surface of a carrier can be modified to include a linking group. The linking group(s) can be introduced by modifying one or more of the targeting agent, therapeutic agent and carrier to include a moiety that forms the linking group when the targeting agent, therapeutic agent and/or carrier are reacted with one another. An exemplary moiety is a double bond. The modified targeting agent, therapeutic agent, and/or carrier can then be reacted with one another using methods known to those skilled in the art, for example, via a Michael reaction (see J. March, Advanced Organic Chemistry 3rd Ed., pp. 711-712 (1985)). Alternative methods for attaching one or more agents to a carrier through a linking group are known to those skilled in the art and may be identified by routine experimentation informed by the guidance provided herein.

The operative association of the therapeutic agent and carrier, as disclosed herein, may be carried out in a number of different ways known to those skilled in the art. In some embodiments, operative association may take place in solution. In other embodiments, operative association may occur in a solid phase. One method for operatively associating the therapeutic agent and the carrier is by using heat (e.g., heat using a microwave method). In an embodiment, the reaction can be heated up to a temperature in the range of about 100° to about 150°C. In another embodiment, the time the materials are heated ranges from about 5 to about 40 minutes. If desired, the reaction mixture can be cooled to room temperature. These steps may be carried out manually, by automated systems, or by a combination of both.

In some embodiments, the therapeutic agent and targeting agent separately or in combination may be reacted with the carrier to form a mixture. The mixture can be treated (e.g., incubated) under suitable conditions to allow the targeting agent and/or therapeutic agent to become operatively associated with the carrier. If desirable, one of the agents and carrier can be allowed to react before the addition of the other agent. In some embodiments, the targeting agent can be combined with the carrier before the addition of the therapeutic agent. In other embodiments, the therapeutic agent can be combined with the carrier before the addition of the targeting agent. In still other embodiments, the targeting agent and therapeutic agent can be combined at approximately the same time with the carrier.

In some embodiments, one or more of the targeting agent and the therapeutic agent may be operatively associated with a moiety prior to formation of the carrier, wherein the moiety forms a part of the carrier. Exemplary moieties include a lipid, a sugar, protein, or peptide precursor (e.g., an amino acid or a monosaccharide), an amphiphilic molecule, a polymer, or a monomer.

In some embodiments wherein the carrier is a non-cationic polymeric carrier, the targeting agent and/or therapeutic agent can be attached to a monomer that will be used to form part of the non-cationic polymeric carrier. The monomer can then be polymerized using methods known to those skilled in the art to form the non-cationic polymeric carrier. For example, a targeting agent and/or therapeutic agent can be attached to the glutamic acid monomer prior to polymerization. The resulting monomer with the attached targeting agent and/or therapeutic agent can then be polymerized using methods known to those skilled in the art to form the non-cationic polymeric carrier.

In other embodiments, one or more of the targeting agent and the therapeutic agent may be operatively associated with the carrier after it is formed. In some embodiments the carrier may be operatively associated with the targeting agent before it is operatively associated with the therapeutic agent. In other embodiments the carrier may be operatively associated with the targeting agent after it has been operatively associated with the therapeutic agent. In some embodiments, the targeting agent and therapeutic agent can both be electrostatically associated with the carrier. In other embodiments, the targeting agent and therapeutic agent can both be covalently bonded to the carrier. In still other embodiments, one type of agent (e.g., the targeting agent or therapeutic agent) may be electrostatically associated with the carrier and another type of agent (e.g., the therapeutic agent or targeting agent) may be covalently bonded to the carrier.

In some embodiments where a nanomaterial carrier is used, one or more of the targeting agent and the therapeutic agent may be operatively associated with the nanomaterial carrier through emulsion synthesis. In an embodiment, one or more of the targeting agent and the therapeutic agent may be operatively associated with the nanomaterial carrier via a double emulsion solvent diffusion method, such as the water-in-oil-in-water emulsion solvent diffusion method. In another embodiment, an emulsion evaporation method can be used.

Biostructural carriers including a sugar may be operatively associated with the targeting agent and/or therapeutic agent by a variety of methods, including but not limited to, co-grinding, kneading, solid dispersion, solvent evaporation, co-precipitation, spray drying, microwave heating, and freeze drying. Likewise, when the biostructural carrier is a protein or peptide, a variety of methods can be used to operatively associate the biostructural carrier with the targeting agent and/or therapeutic agent. Exemplary methods for operatively associating an agent with a protein or peptide include sonication, cavitation, and ultrasonic emulsification.

The aforementioned reactions can take place at any suitable temperature, such as room temperature. Appropriate solvents, coupling agents, catalysts, and/or buffers as generally known to those skilled in the art and/or as described herein may be used to operatively associate the therapeutic agent, the targeting agent, and the carrier.

In addition, suitable methods known to those skilled in the art can be used to isolate and/or purify the therapeutic composition. For instance, a reaction mixture can be filtered into an acidic water solution. Any precipitate that forms can then be filtered and washed with water. Optionally, the precipitate can be purified by any suitable method known to those skilled in the art. For example, the precipitate can be transferred into acetone and dissolved, and the resulting solution can be filtered again into a sodium bicarbonate solution. If desired, the resulting reaction solution can be dialyzed in water using a cellulose membrane and the polymer can be lyophilized and isolated. After formation of the therapeutic composition, any free amount of targeting agent or therapeutic agent that is not operatively associated with the carrier may also be measured. For example, thin layer chromatography (TLC) may be used to confirm the substantial absence of a free therapeutic agent remaining in the therapeutic composition.

The targeting agent and the therapeutic agent may be operatively associated with the carrier at various positions relative to the carrier. Such positions may be fixed (e.g., at the middle, ends or side chains of a non-cationic polymeric carrier) or relative, e.g., the carrier may exhibit a configuration in a particular medium (such as an aqueous medium) such that it has interior and exterior portions. In an embodiment, one or more of the targeting agent and the therapeutic agent may be operatively associated with an exterior moiety or surface of the carrier. In some embodiments, one or more of the targeting agent and the therapeutic agent may be operatively associated with an interior moiety or surface of the carrier. In an embodiment, one or more of the targeting agent and the therapeutic agent can be at least partially contained within the carrier. In another embodiment, one or more of the targeting agent and the therapeutic agent may be substantially completely contained within the carrier.

In some embodiments wherein the carrier is a non-cationic polymeric carrier, one or more of the targeting agent and the therapeutic agent may be operatively associated with a side chain moiety of the non-cationic polymeric carrier. In other embodiments, one or more of the targeting agent and the therapeutic agent may be operatively associated with an end or terminal recurring unit of the non-cationic polymeric carrier. In yet other embodiments, one or more of the targeting agent and the therapeutic agent may be operatively associated with the middle of the non-cationic polymeric carrier. In still yet other embodiments, one or more of the targeting agent and the therapeutic agent may be operatively associated with the backbone of the non-cationic polymeric carrier.

In some embodiments wherein the carrier is a liposome carrier, one or more of the targeting agent and the therapeutic agent may be partially or completely contained within the lipid bilayer. For example, the targeting agent and/or therapeutic agent may be partially or completely contained between two lipid layers of the lipid bilayer of the liposome carrier.

In some embodiments wherein the carrier is a dendritic carrier, one or more of the targeting agent may be operatively associated with an exterior moiety of a branch of the dendritic carrier. In some embodiments, one or more of the targeting agent and the therapeutic agent may be operatively associated with an interior moiety of a branch or the core of the dendritic carrier.

In some embodiments wherein the carrier is selected from a nanomaterial carrier, a biostructural carrier, and a micelle carrier, one or more of the targeting agent and the therapeutic agent may be operatively associated with an exterior surface of the carrier. In another embodiment, one or more of the targeting agent and the therapeutic agent may be operatively associated with an interior surface of the carrier. As an example, one or more of the targeting agent and the therapeutic agent may be partially or completely encapsulated within the carrier.

In some embodiments, one type of agent (e.g., the therapeutic agent or the targeting agent) may be operatively associated with the carrier at one part, while another type of agent (e.g., the therapeutic agent or the targeting agent) may be operatively associated with the carrier at another part. As an example, the targeting agent may be operatively associated with an exterior moiety or surface of the carrier and the therapeutic agent may be operatively associated with an interior moiety or surface of the carrier. In the alternative, the therapeutic agent can be operatively associated with an exterior surface of the carrier and the targeting agent may be operatively associated with an interior surface or interior moiety, such as a core) of the carrier. In other embodiments, one type of agent (e.g., the therapeutic agent or the targeting agent) may be operatively associated with the carrier at approximately the same part. As an example, both agents may be associated with an interior moiety or surface of the carrier. In the alternative, both agents may be associated with an exterior moiety or surface of the carrier. When one or more of the agents are associated with an interior moiety or surface, each agent may be partially or completely encapsulated within the carrier. Those of ordinary skill in the art will recognize that the location and orientation of association may vary depending on the properties of the specific targeting agent, therapeutic agent, and carrier.

The polymeric non-cationic carriers disclosed herein may be prepared according to a variety of methods, in addition to those described above. Many of the carriers disclosed herein, such as PGA, PGGA, and PLGA, may be commercially available or prepared using methods known to those of ordinary skill in the art.

The liposome carriers disclosed herein may be available, for example, from NDF Corporation under the trade name Coatsome EL. Alternatively, the liposome carriers disclosed herein may be prepared using methods known to those of ordinary skill in the art. *See*, *e.g*., Liposome Technology, 3d Ed., Informa Healthcare, New York (2006), which is incorporated herein by reference in its entirety.

The dendritic carriers may be commercially available from a variety of sources, e.g., Dendritech, Midland, Michigan. In the alternate, the dendritic carriers disclosed herein may be prepared using methods known to those of ordinary skill in the art, including but not limited to, divergent synthesis and convergent synthesis. *See*, *e.g*., J. Peterson, et al., Synthesis and CZE Analysis of PAMAM Dendrimers with an Ethylenediamene Core, Proc. Estonian Acad. Sci. Chem., 50(3):156-166 (2001); C.J. Hawker, J.M.J. Frechet, Preparation of Polymers with Controlled Molecular Architecture, A New Convergent Approach to Dendritic Macromolecules, J. Am. Chem. Soc. 112: 7638-7647 (1990).

The nanomaterial carriers disclosed herein may be prepared according to a variety of methods. Many of the nanomaterial carriers disclosed herein, such as PLGA, gold nanoparticles, and carbon nanotubes, may be commercially available or prepared using methods known to those of ordinary skill in the art. A carbon nanotube, for example, may be obtained from commercial sources or prepared by a variety of methods including, but not limited to, arc discharge, laser ablation, high pressure carbon monoxide, and chemical vapor deposition.

The biostructural carriers disclosed herein may be commercially available or may prepared using methods known to those of ordinary skill in the art. For example, cyclodextrin and many derivatives thereof may be commercially available from a variety of sources, such as Cyclodextrin Technologies Development, Inc., High Springs, Florida. Any of a variety of methods can be used for expressing, purifying, and generating proteins, Methods of expressing, purifying and generating proteins are known in the art, as exemplified in Current Protocols in Protein Science, John Wiley and Sons, Inc. (2007), which is hereby incorporated by reference for the limited purpose of describing methods of expressing, purifying and generating proteins. Methods for preparation of albumin nanoparticles may be known to those skilled in the art. *E.g.* Das, Saikat, et al., Aspirin Loaded Albumin Nanoparticles by Coacervation: Implications in Drug Delivery, Trends Biomater. Artif. Organs, 18(2):203-212 (2005).

The micelle carriers disclosed herein may be commercially available or may prepared using methods known to those of ordinary skill in the art. Those of skill in the art will recognize that many micelle carriers can self-assemble from their starting amphiphilic molecules, such as lipids and/or polymers, at the amphiphilic molecules' critical micelle concentration (cmc) and critical micelle temperature (cmt).

The targeting agents disclosed herein may be commercially available or may be made according to methods known to those of skill in the art. In addition, the therapeutic agents disclosed herein may be prepared according to a variety of methods as known to those of ordinary skill in the art. Certain therapeutic agents, such as paclitaxel, may be commercially available. In some embodiments, the therapeutic agent may include a nucleic acid, such as siRNA, DNA, RNA or an antisense nucleic acid. In some embodiments, a nucleic acid may be specifically adapted to promote degradation of a particular molecule. Such a molecule may be, for example, a tissue inhibitor of metalloproteinases (TIMP) or a molecular chaperone. The molecular chaperone that is inhibited by delivery of a therapeutic agent to a target organ or tissue may be collagen-specific, such as heat shock protein 47 (HSP47). In some embodiments, siRNA may be designed with a particular sequence to recognize HSP47. Those having ordinary skill in the art will recognize that various techniques of designing nucleic acids in this manner are available and that chemically synthesized nucleic acids may be commercially available.

Another embodiment provides a pharmaceutical composition that can include one or more therapeutic compositions described herein, and further including at least one selected from a pharmaceutically acceptable excipient, a pharmaceutical carrier (distinguishable from the carrier described herein), and a diluent. In some embodiments, prodrugs, metabolites, stereoisomers, hydrates, solvates, polymorphs, and pharmaceutically acceptable salts of the compounds disclosed herein (e.g., the therapeutic composition that can include a targeting agent and a therapeutic agent) are provided.

If the manufacture of pharmaceutical formulations involves intimate mixing of the pharmaceutical excipients and the active ingredient in its salt form, then it may be desirable to use pharmaceutical excipients which are non-basic, that is, either acidic or neutral excipients.

In various embodiments, the compositions disclosed herein (e.g., the therapeutic composition that can include a targeting agent and a therapeutic agent) can be used alone, in combination with other compounds disclosed herein, or in combination with one or more other agents active in the therapeutic areas described herein.

In another aspect, the present disclosure relates to a pharmaceutical composition comprising one or more physiologically acceptable surface active agents, pharmaceutical carriers, diluents, excipients, smoothing agents, suspension agents, film forming substances, and coating assistants, or a combination thereof; and a composition (e.g., the therapeutic composition that can include a targeting agent and a therapeutic agent) disclosed herein. Acceptable additional pharmaceutical carriers or diluents for therapeutic use are well known in the pharmaceutical art, and are described, for example, in Remington's Pharmaceutical Sciences, 18th Ed., Mack Publishing Co., Easton, PA (1990), which is incorporated herein by reference in its entirety. Preservatives, stabilizers, dyes, sweeteners, fragrances, flavoring agents, and the like may be provided in the pharmaceutical composition. For example, sodium benzoate, ascorbic acid and esters of p-hydroxybenzoic acid may be added as preservatives. In addition, antioxidants and suspending agents may be used. In various embodiments, alcohols, esters, sulfated aliphatic alcohols, and the like may be used as surface active agents; sucrose, glucose, lactose, starch, crystallized cellulose, mannitol, light anhydrous silicate, magnesium aluminate, magnesium metasilicate aluminate, synthetic aluminum silicate, calcium carbonate, sodium acid carbonate, calcium hydrogen phosphate, calcium carboxymethyl cellulose, and the like may be used as excipients; magnesium stearate, talc, hardened oil and the like may be used as smoothing agents; coconut oil, olive oil, sesame oil, peanut oil, soya oil may be used as suspension agents or lubricants; cellulose acetate phthalate as a derivative of a carbohydrate such as cellulose or sugar, or methylacetate-methacrylate copolymer as a derivative of polyvinyl may be used as suspension agents; and plasticizers such as ester phthalates and the like may be used as suspension agents.

The term "pharmaceutical composition" refers to a mixture of a composition disclosed herein (e.g., the therapeutic composition that can include a targeting agent and a therapeutic agent) with other chemical components, such as diluents or additional pharmaceutical carriers. The pharmaceutical composition facilitates administration of the compound to an organism. Multiple techniques of administering a pharmaceutical composition exist in the art including, but not limited to, oral, injection, aerosol, parenteral, and topical administration. Pharmaceutical compositions can also be obtained by reacting compounds with inorganic or organic acids such as hydrochloric acid, hydrobromic acid, sulfuric acid, nitric acid, phosphoric acid, methanesulfonic acid, ethanesulfonic acid, p-toluenesulfonic acid, salicylic acid and the like.

The term "pharmaceutical carrier" refers to a second chemical compound, different from and in addition to the carrier, that facilitates the incorporation of a compound into cells or tissues. For example dimethyl sulfoxide (DMSO) is a commonly utilized carrier as it facilitates the uptake of many organic compounds into the cells or tissues of an organism.

The term "diluent" refers to chemical compounds diluted in water that will dissolve the composition of interest (e.g., the therapeutic composition that can include a targeting agent and a therapeutic agent) as well as stabilize the biologically active form of the compound. Salts dissolved in buffered solutions are utilized as diluents in the art. One commonly used buffered solution is phosphate buffered saline because it mimics the salt conditions of human blood. Since buffer salts can control the pH of a solution at low concentrations, a buffered diluent rarely modifies the biological activity of a compound. As used herein, an "excipient" refers to an inert substance that is added to a composition to provide, without limitation, bulk, consistency, stability, binding ability, lubrication, disintegrating ability, etc., to the composition. A "diluent" is a type of excipient.

The term "physiologically acceptable" refers to a pharmaceutical carrier or diluent that does not abrogate the biological activity and properties of the compound.

The pharmaceutical compositions described herein can be administered to a human patient *per se*, or in pharmaceutical compositions where they are mixed with other active ingredients, as in combination therapy, or suitable pharmaceutical carriers or excipient(s). Techniques for formulation and administration of the compounds of the instant application may be found in "Remington's Pharmaceutical Sciences," Mack Publishing Co., Easton, PA, 18th edition, 1990.

Suitable routes of administration may, for example, include oral, rectal, transmucosal, topical, or intestinal administration; parenteral delivery, including intramuscular, subcutaneous, intravenous, intramedullary injections, as well as intrathecal, direct intraventricular, intraperitoneal, intranasal, or intraocular injections. The compounds (e.g., the therapeutic composition that can include a targeting agent and a therapeutic agent) can also be administered in sustained or controlled release dosage forms, including depot injections, osmotic pumps, pills, transdermal (including electrotransport) patches, and the like, for prolonged and/or timed, pulsed administration at a predetermined rate.

The pharmaceutical compositions may be manufactured in a manner that is itself known, e.g., by means of conventional mixing, dissolving, granulating, dragee-making, levigating, emulsifying, encapsulating, entrapping or tabletting processes.

Pharmaceutical compositions may be formulated in any conventional manner using one or more physiologically acceptable pharmaceutical carriers comprising excipients and auxiliaries which facilitate processing of the active compounds into preparations which can be used pharmaceutically. Proper formulation is dependent upon the route of administration chosen. Any of the well-known techniques, pharmaceutical carriers, and excipients may be used as suitable and as understood in the art; *e.g.,* in Remington's Pharmaceutical Sciences, above.

Injectables can be prepared in conventional forms, either as liquid solutions or suspensions, solid forms suitable for solution or suspension in liquid prior to injection, or as emulsions. Suitable excipients are, for example, water, saline, dextrose, mannitol, lactose, lecithin, albumin, sodium glutamate, cysteine hydrochloride, and the like. In addition, if desired, the injectable pharmaceutical compositions may contain minor amounts of nontoxic auxiliary substances, such as wetting agents, pH buffering agents, and the like. Physiologically compatible buffers include, but are not limited to, Hanks's solution, Ringer's solution, or physiological saline buffer. If desired, absorption enhancing preparations may be utilized.

For transmucosal administration, penetrants appropriate to the barrier to be permeated may be used in the formulation.

Pharmaceutical formulations for parenteral administration, *e.g*., by bolus injection or continuous infusion, include aqueous solutions of the active compounds in water-soluble form. Additionally, suspensions of the active compounds may be prepared as appropriate oily injection suspensions. Aqueous injection suspensions may contain substances which increase the viscosity of the suspension, such as sodium carboxymethyl cellulose, sorbitol, or dextran. Optionally, the suspension may also contain suitable stabilizers or agents that increase the solubility of the compounds to allow for the preparation of highly concentrated solutions. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, *e.g*., sterile pyrogen-free water, before use.

For oral administration, the composition can be formulated readily by combining the compositions of interest (e.g., the therapeutic composition that can include a targeting agent and a therapeutic agent) with pharmaceutical carriers well known in the art. Such pharmaceutical carriers, which may be used in addition to the carrier(s) disclosed above, enable the compositions to be formulated as tablets, pills, dragees, capsules, liquids, gels, syrups, slurries, suspensions and the like, for oral ingestion by a patient to be treated. Pharmaceutical preparations for oral use can be obtained by combining the active compounds with solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethylcellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate. Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. All formulations for oral administration should be in dosages suitable for such administration.

For buccal administration, the compositions may take the form of tablets or lozenges formulated in conventional manner.

For administration by inhalation, the composition can be conveniently delivered in the form of an aerosol spray presentation from pressurized packs or a nebulizer, with the use of a suitable propellant, *e.g*., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of, *e.g*., gelatin for use in an inhaler or insufflator may be formulated containing a powder mix of the compound and a suitable powder base such as lactose or starch.

Further disclosed herein are various pharmaceutical compositions well known in the pharmaceutical art for uses that include intraocular, intranasal, and intraauricular delivery. Suitable penetrants for these uses are generally known in the art. Such suitable pharmaceutical formulations are most often and preferably formulated to be sterile, isotonic and buffered for stability and comfort. Pharmaceutical compositions for intranasal delivery may also include drops and sprays often prepared to simulate in many respects nasal secretions to ensure maintenance of normal ciliary action. As disclosed in Remington's Pharmaceutical Sciences, 18th Ed., Mack Publishing Co., Easton, PA (1990), which is incorporated herein by reference in its entirety, and well-known to those skilled in the art, suitable formulations are most often and preferably isotonic, slightly buffered to maintain a pH of 5.5 to 6.5, and most often and preferably include antimicrobial preservatives and appropriate drug stabilizers. Pharmaceutical formulations for intraauricular delivery include suspensions and ointments for topical application in the ear. Common solvents for such aural formulations include glycerin and water.

The compositions may also be formulated in rectal compositions such as suppositories or retention enemas, *e.g*., containing conventional suppository bases such as cocoa butter or other glycerides.

In addition to the formulations described previously, the compositions may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

For hydrophobic compounds, a suitable pharmaceutical carrier may be a cosolvent system comprising benzyl alcohol, a nonpolar surfactant, a water-miscible organic polymer, and an aqueous phase. A common cosolvent system used is the VPD co-solvent system, which is a solution of 3% w/v benzyl alcohol, 8% w/v of the nonpolar surfactant Polysorbate 80™, and 65% w/v polyethylene glycol 300, made up to volume in absolute ethanol. Naturally, the proportions of a co-solvent system may be varied considerably without destroying its solubility and toxicity characteristics. Furthermore, the identity of the co-solvent components may be varied: for example, other low-toxicity nonpolar surfactants may be used instead of POLYSORBATE 80™; the fraction size of polyethylene glycol may be varied; other biocompatible polymers may replace polyethylene glycol, *e.g*., polyvinyl pyrrolidone; and other sugars or polysaccharides may substitute for dextrose.

Disclosed herein are methods for treating a condition characterized by abnormal fibrosis, which may include administering a therapeutically effective amount of therapeutic compositions as described herein. Conditions characterized by abnormal fibrosis may include cancer and/or a fibrotic disease. Types of cancer that may be treated or ameliorated by a therapeutic composition described herein include, but are not limited to, lung cancer, pancreatic cancer, breast cancer, liver cancer, stomach cancer, and colon cancer. In an embodiment, the cancer that may be treated or ameliorated is pancreatic cancer. In another embodiment, the cancer that may be treated or ameliorated is lung cancer. Types of fibrotic disease that may be treated or ameliorated by a therapeutic composition described herein include, but are not limited to, hepatic fibrosis, hepatic cirrhosis, pancreatitis, pancreatic fibrosis, cystic fibrosis, vocal cord scarring, vocal cord mucosal fibrosis, laryngeal fibrosis, pulmonary fibrosis, idiopathic pulmonary fibrosis, cystic fibrosis, myelofibrosis, retroperitoneal fibrosis, and nephrogenic systemic fibrosis. In an embodiment, the condition that may be treated or ameliorated is hepatic fibrosis.

The compositions or pharmaceutical compositions described herein may be administered to the subject by any suitable means. Non-limiting examples of methods of administration include, among others, (a) administration though oral pathways, which administration includes administration in capsule, tablet, granule, spray, syrup, or other such forms; (b) administration through non-oral pathways such as rectal, vaginal, intraurethral, intraocular, intranasal, or intraauricular, which administration includes administration as an aqueous suspension, an oily preparation or the like or as a drip, spray, suppository, salve, ointment or the like; (c) administration via injection, subcutaneously, intraperitoneally, intravenously, intramuscularly, intradermally, intraorbitally, intracapsularly, intraspinally, intrasternally, or the like, including infusion pump delivery; (d) administration locally such as by injection directly in the renal or cardiac area, e.g., by depot implantation; as well as (e) administration topically; as deemed appropriate by those of skill in the art for bringing the active compound into contact with living tissue.

Pharmaceutical compositions suitable for administration include compositions where the active ingredients are contained in an amount effective to achieve its intended purpose. The therapeutically effective amount of the compounds disclosed herein required as a dose will depend on the route of administration, the type of animal, including human, being treated, and the physical characteristics of the specific animal under consideration. The dose can be tailored to achieve a desired effect, but will depend on such factors as weight, diet, concurrent medication and other factors which those skilled in the medical arts will recognize. More specifically, a therapeutically effective amount means an amount of compound effective to prevent, alleviate or ameliorate symptoms of disease or prolong the survival of the subject being treated. Determination of a therapeutically effective amount is well within the capability of those skilled in the art, especially in light of the detailed disclosure provided herein.

As will be readily apparent to one skilled in the art, the useful *in vivo* dosage to be administered and the particular mode of administration will vary depending upon the age, weight and mammalian species treated, the particular compounds employed, and the specific use for which these compounds are employed. The determination of effective dosage levels, that is the dosage levels necessary to achieve the desired result, can be accomplished by one skilled in the art using routine pharmacological methods. Typically, human clinical applications of products are commenced at lower dosage levels, with dosage level being increased until the desired effect is achieved. Alternatively, acceptable *in vitro* studies can be used to establish useful doses and routes of administration of the compositions identified by the present methods using established pharmacological methods.

In non-human animal studies, applications of potential products are commenced at higher dosage levels, with dosage being decreased until the desired effect is no longer achieved or adverse side effects disappear. The dosage may range broadly, depending upon the desired effects and the therapeutic indication. Typically, dosages may be about 10 microgram/kg to about 100 mg/kg body weight, preferably about 100 microgram/kg to about 10 mg/kg body weight. Alternatively dosages may be based and calculated upon the surface area of the patient, as understood by those of skill in the art.

The exact formulation, route of administration and dosage for the pharmaceutical compositions can be chosen by the individual physician in view of the patient's condition. (See *e.g*., Fingl et al. 1975, in "The Pharmacological Basis of Therapeutics", which is hereby incorporated herein by reference in its entirety, with particular reference to Ch. 1, p. 1). Typically, the dose range of the composition administered to the patient can be from about 0.5 to about 1000 mg/kg of the patient's body weight. The dosage may be a single one or a series of two or more given in the course of one or more days, as is needed by the patient. In instances where human dosages for compounds have been established for at least some condition, the dosages will be about the same, or dosages that are about 0.1% to about 500%, more preferably about 25% to about 250% of the established human dosage. Where no human dosage is established, as will be the case for newly-discovered pharmaceutical compositions, a suitable human dosage can be inferred from ED₅₀ or ID₅₀ values, or other appropriate values derived from *in vitro* or *in vivo* studies, as qualified by toxicity studies and efficacy studies in animals.

It should be noted that the attending physician would know how to and when to terminate, interrupt, or adjust administration due to toxicity or organ dysfunctions. Conversely, the attending physician would also know to adjust treatment to higher levels if the clinical response were not adequate (precluding toxicity). The magnitude of an administrated dose in the management of the disorder of interest will vary with the severity of the condition to be treated and to the route of administration. The severity of the condition may, for example, be evaluated, in part, by standard prognostic evaluation methods. Further, the dose and perhaps dose frequency, will also vary according to the age, body weight, and response of the individual patient. A program comparable to that discussed above may be used in veterinary medicine.

Although the exact dosage will be determined on a drug-by-drug basis, in most cases, some generalizations regarding the dosage can be made. The daily dosage regimen for an adult human patient may be, for example, an oral dose of about 0.1 mg to 2000 mg of each active ingredient, preferably about 1 mg to about 500 mg, e.g. 5 to 200 mg. In other embodiments, an intravenous, subcutaneous, or intramuscular dose of each active ingredient of about 0.01 mg to about 100 mg, preferably about 0.1 mg to about 60 mg, e.g. about 1 to about 40 mg is used. In cases of administration of a pharmaceutically acceptable salt, dosages may be calculated as the free base. In some embodiments, the composition is administered 1 to 4 times per day. Alternatively the compositions may be administered by continuous intravenous infusion, preferably at a dose of each active ingredient up to about 1000 mg per day. As will be understood by those of skill in the art, in certain situations it may be necessary to administer the compounds disclosed herein in amounts that exceed, or even far exceed, the above-stated, preferred dosage range in order to effectively and aggressively treat particularly aggressive diseases or infections. In some embodiments, the compounds will be administered for a period of continuous therapy, for example for a week or more, or for months or years.

Dosage amount and interval may be adjusted individually to provide plasma levels of the active moiety which are sufficient to maintain the modulating effects, or minimal effective concentration (MEC). The MEC will vary for each compound but can be estimated from in vitro data. Dosages necessary to achieve the MEC will depend on individual characteristics and route of administration. However, HPLC assays or bioassays can be used to determine plasma concentrations.

Dosage intervals can also be determined using MEC value. Compositions should be administered using a regimen which maintains plasma levels above the MEC for 10-90% of the time, preferably between 30-90% and most preferably between 50-90%.

In cases of local administration or selective uptake, the effective local concentration of the drug may not be related to plasma concentration.

The amount of composition administered may be dependent on the subject being treated, on the subject's weight, the severity of the affliction, the manner of administration and the judgment of the prescribing physician.

Compositions disclosed herein (e.g., the therapeutic composition that can include a targeting agent and a therapeutic agent) can be evaluated for efficacy and toxicity using known methods. For example, the toxicology of a particular compound, or of a subset of the compounds, sharing certain chemical moieties, may be established by determining *in vitro* toxicity towards a cell line, such as a mammalian, and preferably human, cell line. The results of such studies are often predictive of toxicity in animals, such as mammals, or more specifically, humans. Alternatively, the toxicity of particular compounds in an animal model, such as mice, rats, rabbits, or monkeys, may be determined using known methods. The efficacy of a particular compound may be established using several recognized methods, such as *in vitro* methods, animal models, or human clinical trials. Recognized *in vitro* models exist for nearly every class of condition, including but not limited to cancer, cardiovascular disease, and various immune dysfunction. Similarly, acceptable animal models may be used to establish efficacy of chemicals to treat such conditions. When selecting a model to determine efficacy, the skilled artisan can be guided by the state of the art to choose an appropriate model, dose, and route of administration, and regime. Of course, human clinical trials can also be used to determine the efficacy of a compound in humans.

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration. The pack or dispenser may also be accompanied with a notice associated with the container in form prescribed by a governmental agency regulating the manufacture, use, or sale of pharmaceuticals, which notice is reflective of approval by the agency of the form of the drug for human or veterinary administration. Such notice, for example, may be the labeling approved by the U.S. Food and Drug Administration for prescription drugs, or the approved product insert. Compositions comprising a compound formulated in a compatible pharmaceutical carrier may also be prepared, placed in an appropriate container, and labeled for treatment of an indicated condition.

### EXAMPLES

The following examples are provided for the purposes of further describing the embodiments described herein, and do not limit the scope of the invention.

### EXAMPLE 1

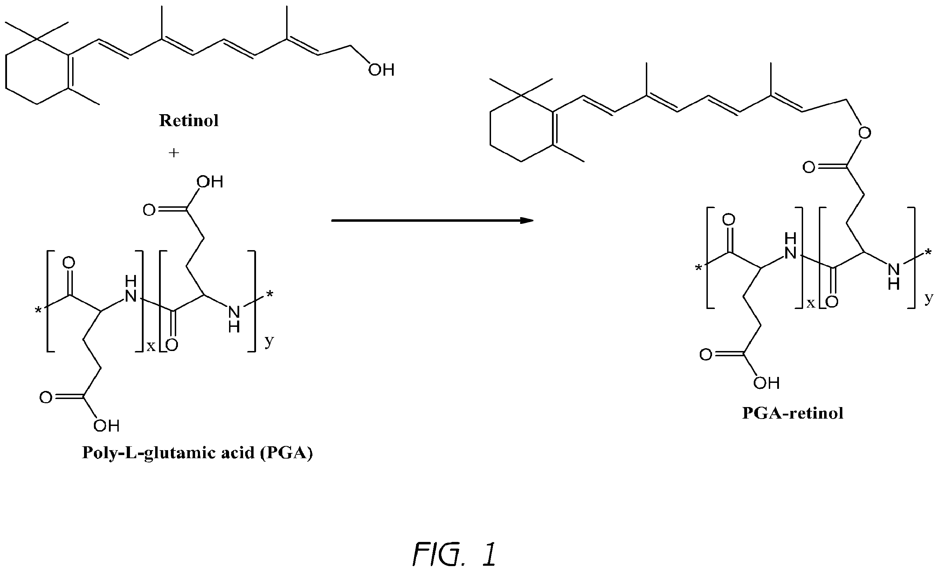

A poly-L-glutamic acid (PGA)-retinol composition was prepared according to the general scheme illustrated in Figure 1 as follows: Poly-L-glutamic acid (PGA, 200 mg) was dissolved in DMF (10 mL). Retinol (10 mg) and EDC (30 mg) and DMAP (5 mg) were added into the solution. The solution was placed under a microwave condition for 5 minutes. The reaction mixture was poured into 0.2N HCl solution. White precipitate was isolated by centrifugation. The precipitate was re-dissolved in 0.5 M sodium bicarbonate solution. The solution was placed under dialysis against water. The product PGA-retinol was lyophilized. Identity of the product was confirmed by ¹H-NMR. The same product, PGA-retinol, was also obtained and confirmed by ¹H-NMR starting with 5 mg of retinol.

### EXAMPLE 2

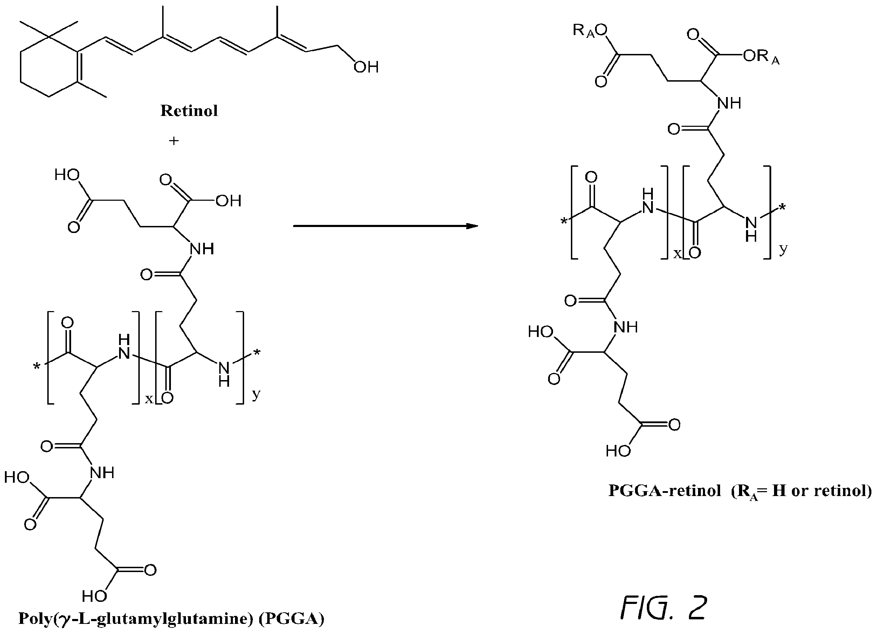

A poly(L-γ-glutamylglutamine) (PGGA)-retinol composition was prepared according to the general scheme illustrated in Figure 2 as follows: Poly(L-γ-glutamylglutamine) (PGGA, 200 mg) was dissolved in DMF (10 mL). Retinol (5 mg) and EDC (30 mg) and DMAP (5 mg) were added into the solution. The solution was placed under a microwave condition for 5 minutes. The reaction mixture was poured into 0.2 N HCl solution. White precipitate was isolated by centrifugation. The precipitate was re-dissolved in 0.5 M sodium bicarbonate solution. The solution was placed under dialysis against water. The PGGA-retinol product was lyophilized. The identity of the product was confirmed by ¹H-NMR.

### EXAMPLE 3

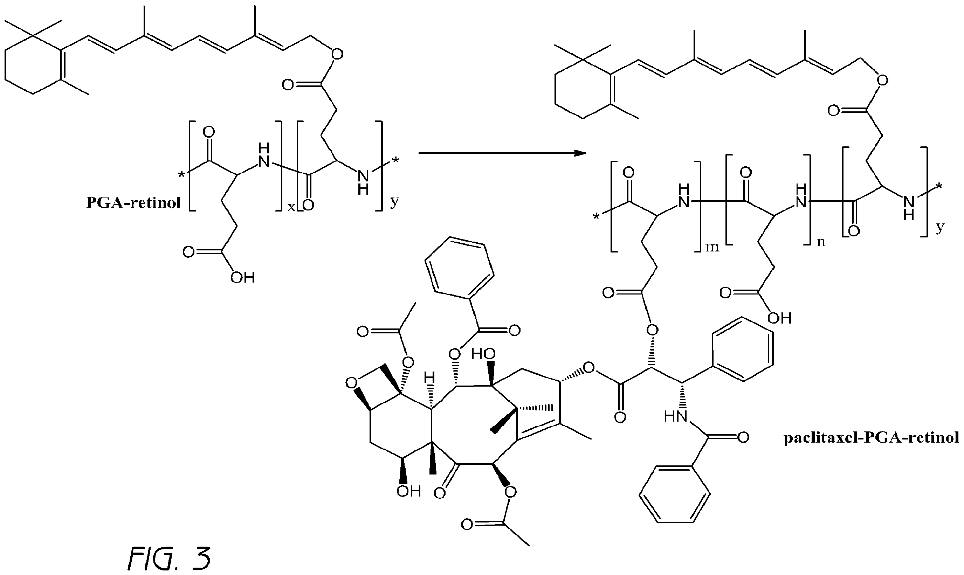

A paclitaxel-PGA-retinol composition was prepared according to the general scheme illustrated in Figure 3 as follows: PGA-retinol from Example 1 (150 mg) was acidified with 0.2 N HCl solution. The acid form of PGA-retinol was isolated by centrifugation and lyophilized. The acid form (100 mg) was then dissolved in DMF (10 mL). Paclitaxel (10 mg), EDC (30 mg) and DMAP (5 mg) were added into the solution. The solution was placed under a microwave condition for 5 minutes. The reaction mixture was poured into 0.2 N HCl solution. White precipitate was isolated by centrifugation. The precipitate was re-dissolved in 0.5 M sodium bicarbonate solution. The solution was placed under dialysis against water. The product was lyophilized. The identity of the product was confirmed by ¹H-NMR.

### EXAMPLE 4

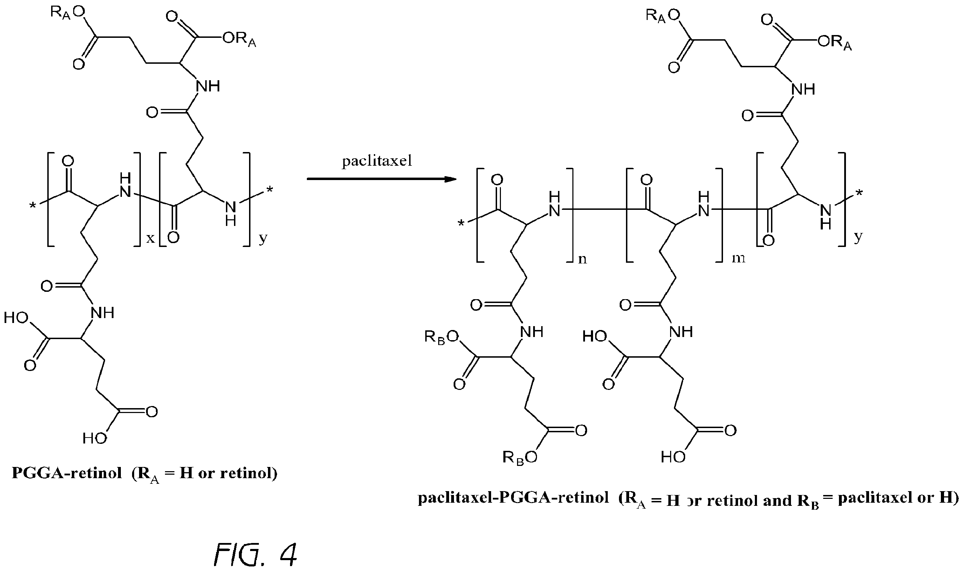

A paclitaxel-PGGA-retinol composition was prepared according to the general scheme illustrated in Figure 4 as follows: PGGA-retinol from Example 2 (150 mg) was acidified with 0.2 N HCl solution. The acid form of PGGA-retinol was isolated by centrifugation and lyophilized. The acid form (100 mg) was then dissolved in DMF (10 mL). Paclitaxel (10 mg), EDC (30 mg) and DMAP (5 mg) were added into the solution. The solution was placed under a microwave condition for 5 minutes. The reaction mixture was poured into 0.2 N HCl solution. White precipitate was isolated by centrifugation. The precipitate was re-dissolved in 0.5 M sodium bicarbonate solution. The solution was placed under dialysis against water. The product was lyophilized. The identity of the product was confirmed by ¹H-NMR.

### EXAMPLE 5

### Synthesis of Texas Red-Poly(L-glutamic acid)-cholesterol (TR-PGA-cholesterol)

Poly(L-glutamic acid) (PGA, 99.7 mg) was placed into a 50 mL round flask. Anhydrous DMF (15mL) was added to the flask and the suspension was stirred for 30 minutes. Cholesterol (5.9 mg), EDC (10.7 mg), and a trace amount of DMAP were added. The mixture was stirred for 40 hours. Texas Red (1 mg in 1 mL DMF), EDC (300 µL, 5mg/mL DMF), and HOBt (300 µL, 1mg/mL DMF) were added to the reaction mixture. The mixture was stirred for 15 hours. The reaction mixture was then poured into 0.2 N HCl aqueous solution (75 mL). The resulting mixture was transferred to centrifuge tubes and centrifuged. The supernatant was discarded. The solid was dissolved in 0.5N NaHCO₃ aqueous solution (approximately 60 mL). The solution was dialyzed against deionized water, filtered through a 0.45 µm cellulose acetate syringe filter and lyophilized. TR-PGA-cholesterol (90 mg) was obtained, and characterized by ¹H-NMR and UV-Vis spectroscopy.

### EXAMPLE 6

### Synthesis of Texas Red-Poly(L-glutamic acid)-retinoid (TR-PGA-retinoid)

Poly(L-glutamic acid)(PGA, 95.6 mg) was placed into a 50mL round flask. Anhydrous DMF (15mL) was added into the flask, and the suspension was stirred for 30 minutes. Retinol (5.5 mg), EDC (12.7 mg), and a trace amount of DMAP were added. The mixture was stirred for 40 hours. Texas Red (1 mg in 1 mL DMF), EDC (300 µL, 5mg/mL DMF), and HOBt (300 µL, 1mg/mL DMF) were added to the reaction mixture. The mixture was stirred for 15 hours. The reaction mixture was then poured into 0.2 N HCl aqueous solution (75 mL). The resulting mixture was transferred to centrifuge tubes and centrifuged. The supernatant was discarded. The solid was dissolved in 0.5 N NaHCO₃ aqueous solution (approximately 60 mL). The solution was then dialyzed against deionized water, filtered through a 0.45 µm cellulose acetate syringe filter and lyophilized. TR-PGA-retinoid (93 mg) was obtained, and characterized by ¹H-NMR and UV-Vis spectroscopy.

### EXAMPLE 7

### Synthesis of Texas Red-Poly(L-γ-glutamylglutamine)-Retinoid (TR-PGGA-Retinoid)

Poly(L-γ-glutamylglutamine) (PGGA, 95.5 mg) was placed into a 50mL round flask. Anhydrous DMF (6 mL) was added into the flask, and the suspension was stirred for 30 minutes. Retinol (5.0mg), EDC (16.3 mg), and a trace amount of DMAP were added. The mixture was stirred for 40 hours. Texas Red (1 mg in 1 mL DMF), EDC (300 µL, 5mg/mL DMF), and HOBt (300 µL, 1mg/mL DMF) were added to the reaction mixture. The mixture was stirred for 15 hours. The reaction mixture was then poured into 0.2 N HCl aqueous solution (75 mL). The resulting mixture was transferred to centrifuge tubes and centrifuged. The supernatant was discarded. The solid was dissolved in 0.5N NaHCO₃ aqueous solution (approximately 60 mL) The solution was dialyzed against deionized water, filtered through a 0.45µm cellulose acetate syringe filter and lyophilized. TR-PGGA-retinoid (91 mg) was obtained, and characterized by ¹H-NMR and UV-Vis spectroscopy.

### EXAMPLE 8

### Synthesis of Texas Red-Dextran-Oleic Acid (TR-Dextran-Oleic acid)

The following compound concentrations were prepared in DMF:
[EDC and HOBt] in DMF = 5 mg/mL
[TR-Dextran-Lys] in DMF = 5 mg/mL
[Oleic acid] in DMF = 3.2 mg/mL

A solution of oleic acid (25 µL, 3.2 mg/mL in DMF) was added to a solution of EDC and HOBt (500 µL, 5 mg/mL in DMF). The reaction was stirred for 25 minutes. A suspension of TR-Dextran-Lys (1000 µL, 5 mg/mL in DMF) was added into the reaction mixture and it was stirred for 10 minutes. Water (1 mL) was added and the reaction mixture was stirred for 15 hours. The solution was dialyzed against deionized water, filtered through a 0.22 µm cellulose acetate syringe filter and lyophilized. TR-Dextran-oleic acid (5 mg) was obtained, and characterized by UV-Vis spectroscopy.

### EXAMPLE 9

### Synthesis of Texas Red-Dextran-Retinoid (TR-Dextran-Retinoid)

The following compound concentrations were prepared in DMF:
[EDC and HOBt] in DMF = 5 mg/mL
[TR-Dextran-Lys] in DMF = 5 mg/mL
[Retinoic acid] in DMF = 1.2 mg/mL

A solution of retinoic acid (100 µL, 1.2 mg/mL in DMF) was added to a solution of EDC and HOBt (500 µL, 5 mg/mL in DMF). The reaction was stirred for 25 minutes. A suspension of TR-Dextran-Lys (1000 µL, 5 mg/mL in DMF) was added into the reaction mixture and it was stirred for 10 minutes. Water (1 mL) was added and the reaction mixture was stirred for 15 hours. The solution was dialyzed against deionized water. The solution was filtered through a 0.22 µm cellulose acetate syringe filter and lyophilized. TR-Dextran-retinoid (5 mg) was obtained, and characterized by UV-Vis spectroscopy.

### EXAMPLE 10

### HSC-T6 cells uptake of retinoid compounds

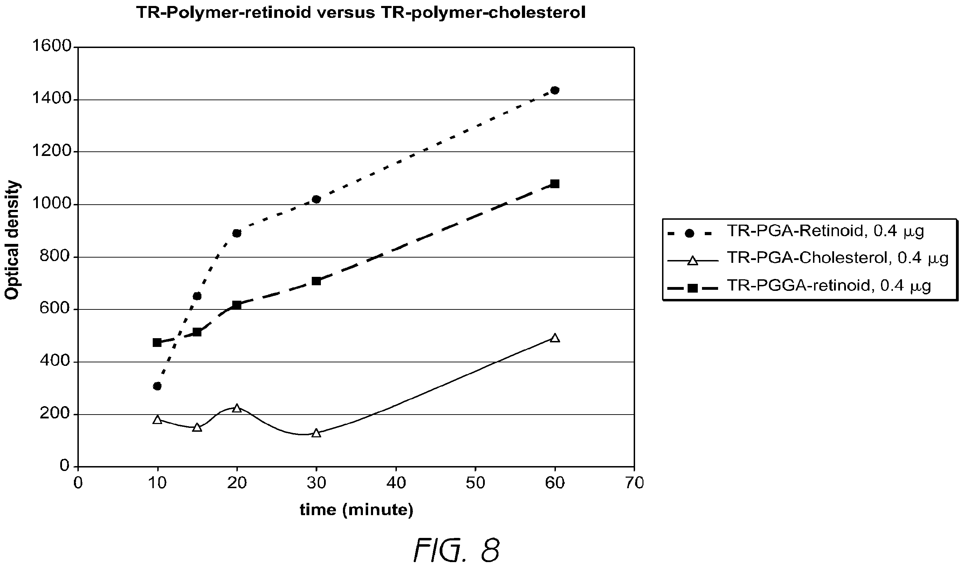

HSC-T6 cells, which express a vitamin A binding protein receptor, were seeded one day prior to experiment in a 96-well plate (100 µL culture medium per well). TR-PGA-cholesterol, TR-PGA-retinoid, TR-PGGA-retinoid, TR-Dextran-oleic acid, and TR-Dextran-retinoid, as prepared in Examples 5-9, were dissolved in water to make approximately 2-4 mg/mL stock solutions. The solutions were diluted with culture medium and incubated for 15 minutes at room temperature. 15 µL was added to the cells. After incubating the cells in solution, the culture medium was removed. Cells were washed once with DPBS and fresh culture medium was added (100 µL culture medium per well). Absorbance (excitation and emission wavelengths were 560 nm and 590 nm, respectively) was read by a BioTek FLx800 96-well plate fluorescence reader and recorded. The results are shown in Figures 8-9.

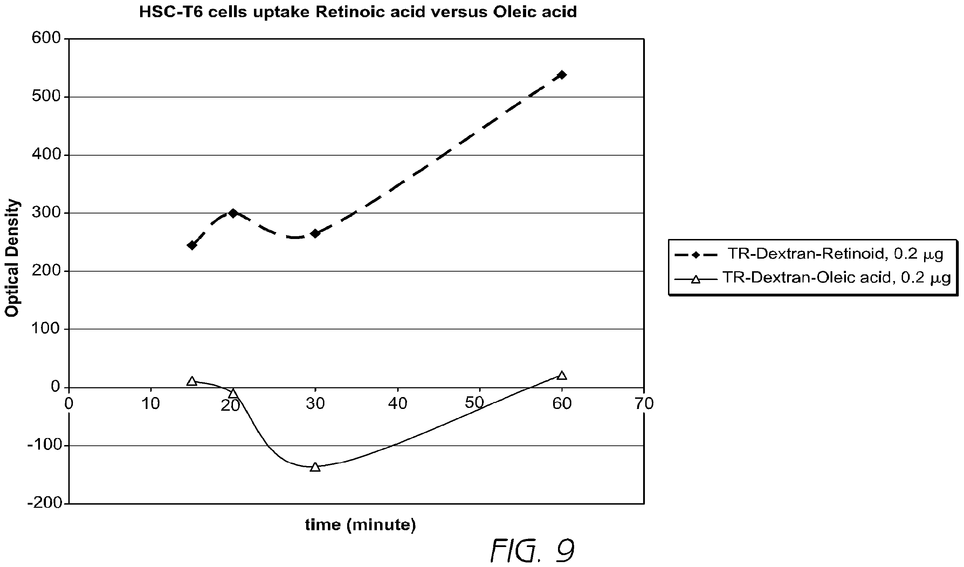

Figure 8 compares the cell uptake of Texas Red-non-cationic polymeric carrier-retinoid with the cell uptake of Texas Red-non-cationic polymeric carrier-cholesterol. Greater absorbance indicates greater optical density and greater cell uptake. Thus, Figure 8 illustrates that the retinoid compositions resulted in greater cellular uptake than the cholesterol composition. Figure 9 compares the cell uptake of Texas Red-Dextran-retinoid with the cell uptake of Texas Red-Dextran-oleic acid. Similar to Figure 8, greater absorbance indicates greater optical density and greater cell uptake. Thus, Figure 9 illustrates that the retinoid composition resulted in greater cellular uptake than the oleic acid composition.

## Claims

1. A therapeutic composition comprising:
a carrier selected from the group consisting of a non-cationic polymeric carrier, a liposome carrier, a dendritic carrier, a nanomaterial carrier, a biostructural carrier, and a micelle carrier;
a targeting agent operatively associated with the carrier, wherein the targeting agent comprises a retinoid; and
a therapeutic agent operatively associated with the carrier, wherein the therapeutic agent exhibits a therapeutic activity upon delivery to a target organ or tissue, and wherein the therapeutic activity is selected from the group consisting of inhibiting fibrosis within the target organ or tissue and inhibiting the growth of a cancer cell within the target organ or tissue.

2. The therapeutic composition of Claim 1, wherein the retinoid is selected from the group consisting of retinol, retinal and retinoic acid, or wherein the retinoid is selected from the group consisting of all-trans retinol, all-trans retinoic acid, retinyl palmitate, 11-cis-retinal, and 13-cis-retinoic acid.

3. The therapeutic composition of any one of Claims 1-2, wherein the target organ is selected from the group consisting of liver, pancreas, kidney, lung, esophagus, larynx, bone marrow, and brain.

4. The therapeutic composition of any one of Claims 1-3, wherein the targeting agent provides an increase in the delivery selectivity of the therapeutic composition, upon delivery to the target organ or tissue, that is at least about two-fold as compared to that of an otherwise comparable therapeutic composition without the targeting agent.

5. The therapeutic composition of any one of Claims 1-4,
wherein the carrier is a non cationic polymeric carrier that comprises a recurring unit of Formula (I): 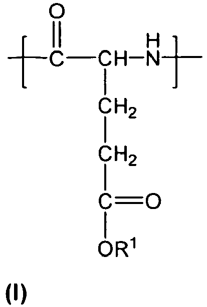 wherein:
each R¹ is selected from the group consisting of hydrogen, ammonium, and an alkali metal;
or wherein the carrier is a non-cationic polymeric carrier that comprises a recurring unit of Formula (II): 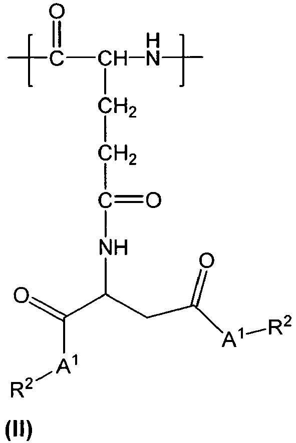 wherein:
each A¹ is independently selected from the group consisting of oxygen and NR⁴;
each R² is independently selected from the group consisting of hydrogen, an optionally substituted C₁₋₁₀ alkyl, an optionally substituted C₆₋₂₀ aryl, ammonium, and an alkali metal; and
R⁴ is selected from the group consisting of hydrogen and C₁₋₄ alkyl;
or wherein the carrier is a non-cationic polymeric carrier that comprises a recurring unit of Formula (III): 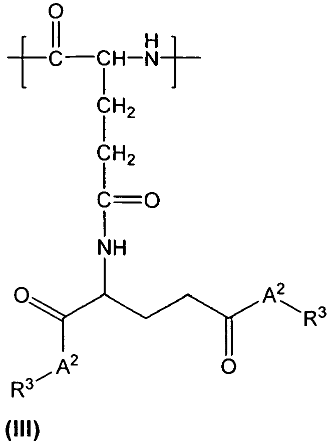 wherein:
each A² is independently selected from the group consisting of oxygen and NR⁵;
each R³ is independently selected from the group consisting of hydrogen, an optionally substituted C₁₋₁₀ alkyl, an optionally substituted C₆₋₂₀ aryl, ammonium, and an alkali metal; and
R⁵ is selected from the group consisting of hydrogen and C₁₋₄ alkyl;
or wherein the non-cationic polymeric carrier comprises a recurring unit selected from the group consisting of Formula (IV) and Formula (V):

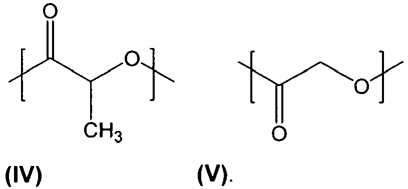

6. The therapeutic composition of any of Claims 1-4, wherein the carrier is a non-cationic polymeric carrier that comprises poly-glutamic acid (PGA).

7. The therapeutic composition of any of Claims 1-4, wherein the carrier is a non-cationic polymeric carrier that comprises one or more of poly(γ-L-glutamylglutamine) (PGGA), poly(γ-L-aspartylglutamine) (PGAA), and poly(lactic-acid-co-glycolic acid (PLGA).

8. The therapeutic composition of any one of Claims 5-7, wherein the non-cationic polymeric carrier comprises a microparticle or a nanoparticle.

9. The therapeutic composition of any one of Claims 1-8, wherein the therapeutic agent substantially inhibits the activation of stellate cells upon delivery to the target organ or tissue.

10. The therapeutic composition of any one of Claims 1-9, wherein the therapeutic agent comprises an agent selected from the group consisting of a nucleic acid and an anti-cancer agent.

11. The therapeutic composition of Claim 10, wherein the therapeutic agent, upon delivery to the target organ or tissue, substantially inhibits the activity of HSP47.

12. The therapeutic composition of any one of Claims 10-11, wherein the therapeutic agent is selected from the group consisting of siRNA, DNA, RNA, and an antisense nucleic acid.

13. Use of a therapeutically effective amount of one or more of the therapeutic compositions of any one of Claims 1-12 in the preparation of a medicament for treating a condition characterized at least in part by abnormal fibrosis.

14. A therapeutic composition of any one of Claims 1-12 for use in treating a condition characterized at least in part by abnormal fibrosis.

15. The use of Claim 13 or the therapeutic composition of Claim 14, wherein the condition is selected from the group consisting of a fibrotic disease and cancer.

## Patentansprüche

1. Therapeutische Zusammensetzung, Folgendes umfassend:
einen Träger, ausgewählt aus der Gruppe bestehend aus einem nicht-kationischen polymeren Träger, einem Liposomträger, einem dendritischen Träger, einem Nanomaterialträger, einem Biostrukturträger und einem Mizellenträger;
ein Targeting-Mittel, das mit dem Träger wirkverbunden ist, wobei das Targeting-Mittel ein Retinoid umfasst; und
ein Therapeutikum, das mit dem Träger wirkverbunden ist, wobei das Therapeutikum nach der Verabreichung an ein Zielorgan oder -gewebe eine therapeutische Aktivität aufweist und
wobei die therapeutische Aktivität ausgewählt ist aus der Gruppe bestehend aus dem Hemmen von Fibrose in dem Zielorgan oder -gewebe und dem Hemmen des Wachstums einer Krebszelle in dem Zielorgan oder -gewebe.

2. Therapeutische Zusammensetzung nach Anspruch 1, wobei das Retinoid ausgewählt ist aus der Gruppe bestehend aus Retinol, Retinal und Retinolsäure oder wobei das Retinoid ausgewählt ist aus der Gruppe bestehend aus all-trans-Retinol, all-trans-Retinolsäure, Retinylpalmitat, 11-cis-Retinal und 13-cis-Retinolsäure.

3. Therapeutische Zusammensetzung nach einem der Ansprüche 1-2, wobei das Zielorgan ausgewählt ist aus der Gruppe bestehend aus Leber, Pankreas, Niere, Lunge, Ösophagus, Larynx, Knochenmark und Hirn.

4. Therapeutische Zusammensetzung nach einem der Ansprüche 1-3, wobei das Targeting-Mittel nach der Verabreichung an das Zielorgan oder -gewebe für eine Erhöhung in der Verabreichungsselektivität der therapeutischen Zusammensetzung sorgt, die verglichen mit der einer anderweitig vergleichbaren therapeutischen Zusammensetzung ohne das Targeting-Mittel wenigstens etwa das Zweifache ist.

5. Therapeutische Zusammensetzung nach einem der Ansprüche 1-4,
wobei der Träger ein nicht-kationischer polymerer Träger ist, der eine wiederkehrende Einheit mit der Formel (I) umfasst: 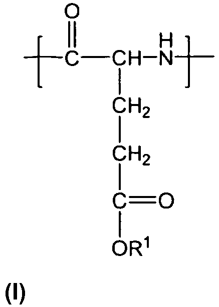 wobei:
jedes R¹ ausgewählt ist aus der Gruppe bestehend aus Wasserstoff, Ammonium und einem Alkalimetall;
oder wobei der Träger ein nicht-kationischer polymerer Träger ist, der eine wiederkehrende Einheit mit der Formel (II) umfasst: 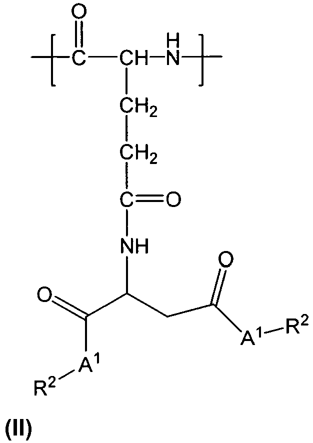 wobei:
jedes A¹ unabhängig ausgewählt ist aus der Gruppe bestehend aus Sauerstoff und NR⁴;
jedes R² unabhängig ausgewählt ist aus der Gruppe bestehend aus Wasserstoff, einem optional substituierten C₁₋₁₀-Alkyl, einem optional substituierten C₆₋₂₀-Aryl, Ammonium und
einem Alkalimetall; und
R⁴ ausgewählt ist aus der Gruppe bestehend aus Wasserstoff und C₁₋₄-Alkyl;
oder wobei der Träger ein nicht-kationischer polymerer Träger ist, der eine wiederkehrende Einheit mit der Formel (III) umfasst: 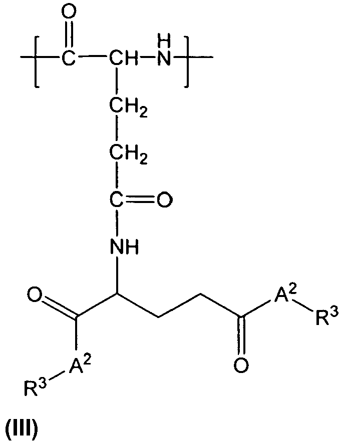 wobei:
jedes A² unabhängig ausgewählt ist aus der Gruppe bestehend aus Sauerstoff und NR⁵;
jedes R³ unabhängig ausgewählt ist aus der Gruppe bestehend aus Wasserstoff, einem optional substituierten C₁₋₁₀-Alkyl, einem optional substituierten C₆₋₂₀-Aryl, Ammonium und einem Alkalimetall; und
R⁵ ausgewählt ist aus der Gruppe bestehend aus Wasserstoff und C₁₋₄-Alkyl;
oder wobei der nicht-kationische polymere Träger eine wiederkehrende Einheit, ausgewählt aus der Gruppe bestehend aus Formel (IV) und Formel (V), umfasst:

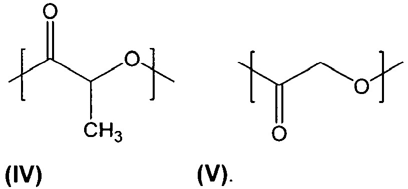

6. Therapeutische Zusammensetzung nach einem der Ansprüche 1-4, wobei der Träger ein nicht-kationischer polymerer Träger ist, der Polyglutaminsäure (PGA) umfasst.

7. Therapeutische Zusammensetzung nach einem der Ansprüche 1-4, wobei der Träger ein nicht-kationischer polymerer Träger ist, der eines oder mehrere von Poly(γ-L-glutamylglutamin) (PGGA), Poly(γ-L-aspartylglutamin) (PGAA) und Poly(milchsäure-co-glycolsäure) (PLGA) umfasst.

8. Therapeutische Zusammensetzung nach einem der Ansprüche 5-7, wobei der nicht-kationische polymere Träger ein Mikropartikel oder ein Nanopartikel umfasst.

9. Therapeutische Zusammensetzung nach einem der Ansprüche 1-8, wobei das Therapeutikum nach der Verabreichung an das Zielorgan oder -gewebe im Wesentlichen die Aktivierung von Sternzellen hemmt.

10. Therapeutische Zusammensetzung nach einem der Ansprüche 1-9, wobei das Therapeutikum ein Mittel, ausgewählt aus der Gruppe bestehend aus einer Nukleinsäure und einem Anti-Krebs-Wirkstoff, umfasst.

11. Therapeutische Zusammensetzung nach Anspruch 10, wobei das Therapeutikum nach der Verabreichung an das Zielorgan oder -gewebe im Wesentlichen die Aktivität von HSP47 hemmt.

12. Therapeutische Zusammensetzung nach einem der Ansprüche 10-11, wobei das Therapeutikum ausgewählt ist aus der Gruppe bestehend aus siRNA, DNA, RNA und einer Antisense-Nukleinsäure.

13. Verwendung einer therapeutisch wirksamen Menge einer oder mehrerer der therapeutischen Zusammensetzungen nach einem der Ansprüche 1-12 beim Herstellen eines Arzneimittels zum Behandeln einer Erkrankung, die wenigstens teilweise durch abnorme Fibrose gekennzeichnet ist.

14. Therapeutische Zusammensetzung nach einem der Ansprüche 1-12 zur Verwendung beim Behandeln einer Krankheit, die wenigstens teilweise durch abnorme Fibrose gekennzeichnet ist.

15. Verwendung nach Anspruch 13 oder therapeutische Zusammensetzung nach Anspruch 14, wobei die Krankheit ausgewählt ist aus der Gruppe bestehend aus einer fibrotischen Erkrankung und Krebs.

## Revendications

1. Composition thérapeutique comprenant :
un vecteur choisi dans le groupe constitué par un vecteur polymère non cationique, un vecteur liposome, un vecteur dendritique, un vecteur nanomatériau, un vecteur biostructural et un vecteur micelle ;
un agent de ciblage opérationnellement associé au vecteur, où l'agent de ciblage comprend un rétinoïde; et
un agent thérapeutique opérationnellement associé au vecteur, où l'agent thérapeutique présente une activité thérapeutique lors de l'administration dans un organe ou tissu cible, et
où l'activité thérapeutique est choisie dans le groupe constitué par l'inhibition de la fibrose dans l'organe ou le tissu cible et l'inhibition de la croissance d'une cellule cancéreuse dans l'organe ou le tissu cible.

2. Composition thérapeutique selon la revendication 1, dans laquelle le rétinoïde est choisi dans le groupe constitué par le rétinol, le rétinal et l'acide rétinoïque, ou dans laquelle le rétinoïde est choisi dans le groupe constitué par le tout-trans-rétinol, l'acide tout-trans-rétinoïque, le palmitate de rétinyle, le 11-cis-rétinal et l'acide 13-cis-rétinoïque.

3. Composition thérapeutique selon l'une quelconque des revendications 1 à 2, dans laquelle l'organe cible est choisi dans le groupe constitué par le foie, le pancréas, le rein, le poumon, l'oesophage, le larynx, la moelle osseuse et le cerveau.

4. Composition thérapeutique selon l'une quelconque des revendications 1 à 3, dans laquelle l'agent de ciblage confère une augmentation de la sélectivité d'administration de la composition thérapeutique, lors de l'administration dans l'organe ou le tissu cible, qui est au moins deux fois plus élevée que celle d'une autre composition thérapeutique comparable ne contenant pas l'agent de ciblage.

5. Composition thérapeutique selon l'une quelconque des revendications 1 à 4,
dans laquelle le vecteur est un vecteur polymère non cationique qui comprend une unité récurrente de Formule (I) : 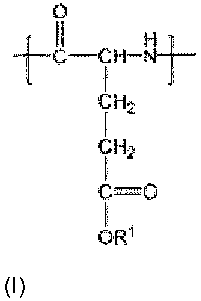 dans laquelle :
chaque R¹ est choisi dans le groupe constitué par l'hydrogène, l'ammonium et un métal alcalin;
ou dans laquelle le vecteur est un vecteur polymère non cationique qui comprend une unité récurrente de Formule (II) : 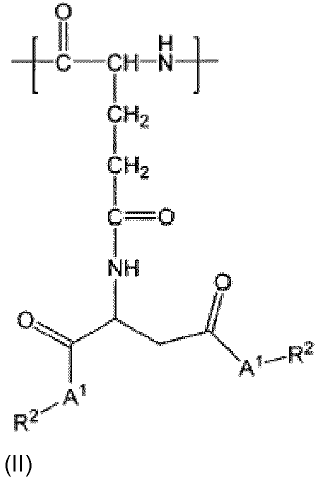 dans laquelle :
chaque A¹ est indépendamment choisi dans le groupe constitué par l'oxygène et NR⁴ ;
chaque R² est indépendamment choisi dans le groupe constitué par l'hydrogène, un alkyle en C₁₋₁₀ facultativement substitué, un aryle en C₆₋₂₀ facultativement substitué, l'ammonium et un métal alcalin ; et
R⁴ est choisi dans le groupe constitué par l'hydrogène et l'alkyle en C₁₋₄ ;
ou dans laquelle le vecteur est un vecteur polymère non cationique qui comprend une unité récurrente de Formule (III) : 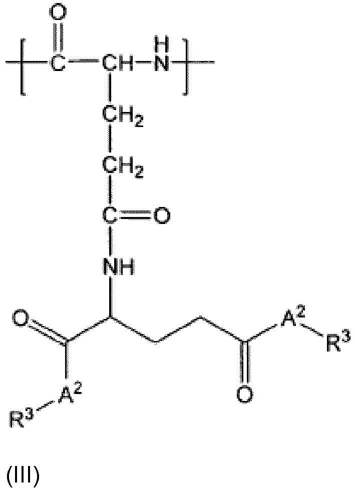 dans laquelle :
chaque A² est indépendamment choisi dans le groupe constitué par l'oxygène et NR⁵ ;
chaque R³ est indépendamment choisi dans le groupe constitué par l'hydrogène, un alkyle en C₁₋₁₀ facultativement substitué, un aryle en C₆₋₂₀ facultativement substitué, l'ammonium et un métal alcalin ; et
R⁵ est choisi dans le groupe constitué par l'hydrogène et l'alkyle en C₁₋₄;
ou dans laquelle le vecteur est un vecteur polymère non cationique qui comprend une unité récurrente choisie dans le groupe constitué par la Formule (IV) et la Formule (V) :

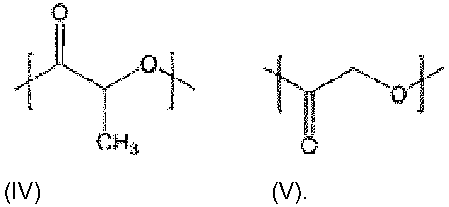

6. Composition thérapeutique selon l'une quelconque des revendications 1 à 4, dans laquelle le vecteur est un vecteur polymère non cationique qui comprend de l'acide polyglutamique (PGA).

7. Composition thérapeutique selon l'une quelconque des revendications 1 à 4, dans laquelle le vecteur est un vecteur polymère non cationique qui comprend un ou plusieurs de la poly(γ-L-glutamylglutamine) (PGGA), de la poly(γ-L-aspartylglutamine (PGAA) et de l'acide poly(lactique-co-glycolique) (PLGA).

8. Composition thérapeutique selon l'une quelconque des revendications 5 à 7, dans laquelle le vecteur polymère non cationique comprend une microparticule ou une nanoparticule.

9. Composition thérapeutique selon l'une quelconque des revendications 1 à 8, dans laquelle l'agent thérapeutique inhibe sensiblement l'activation des cellules stellaires lors de l'administration dans l'organe ou le tissu cible.

10. Composition thérapeutique selon l'une quelconque des revendications 1 à 9, dans laquelle l'agent thérapeutique comprend un agent choisi dans le groupe constitué par un acide nucléique et un agent anticancéreux.

11. Composition thérapeutique selon la revendication 10, dans laquelle l'agent thérapeutique, lors de l'administration dans l'organe ou le tissu cible, inhibe sensiblement l'activité de HSP47.

12. Composition thérapeutique selon l'une quelconque des revendications 10 à 11, dans laquelle l'agent thérapeutique est choisi dans le groupe constitué par l'ARNsi, l'ADN, l'ARN et un acide nucléique antisens.

13. Utilisation d'une quantité thérapeutiquement efficace d'une ou de plusieurs des compositions thérapeutiques selon l'une quelconque des revendications 1 à 12 dans la préparation d'un médicament pour le traitement d'une condition **caractérisée** au moins en partie par une fibrose anormale.

14. Composition thérapeutique selon l'une quelconque des revendications 1 à 12 pour une utilisation dans le traitement d'une condition **caractérisée** au moins en partie par une fibrose anormale.

15. Utilisation selon la revendication 13 ou composition thérapeutique selon la revendication 14, dans laquelle la condition est choisie dans le groupe constitué par une maladie fibrogène ou un cancer.
